# EUROPEAN PATENT SPECIFICATION

(11) **EP 1 586 337 B1**
(45) Date of publication and mention of the grant of the patent: **21.06.2017**
(21) Application number: 05252315.6
(22) Date of filing: 14.04.2005
(51) Int. Cl.: A61F 2/91, A61L 31/16

(54) **The local administration of a combination of rapamycin and 17 beta-estradiol for the treatment of vulnerable plaque**
Lokale Verabreichung einer Kombination von Rapamycin und 17 beta-Estradiol zur Behandlung von vulnerabler Plaque
Administration locale d'une combinaison de rapamycine et 17 beta-estradiol pour le traitement de plaque vulnérable

(30) Priority: 15.04.2004 US 826058
(43) Date of publication of application: 19.10.2005
(60) Divisional application: 15195730.5
(73) Proprietor: Cordis Corporation, Miami Lakes, Florida 33014 (US)
(72) Inventor: Falotico, Robert, Belle Mead, NJ 08502 (US)
(74) Representative: Prock, Thomas

(56) References cited:
- EP-A- 1 362 602
- EP-A- 1 586 338
- WO-A-01/97809

## Description

### BACKGROUND OF THE INVENTION

### Field of the Invention

The present invention relates to intraluminal medical devices comprising a stent for the local delivery of drug combinations for the prevention and treatment of atherosclerotic vulnerable plaque. The drugs may be utilized to promote healing and endothelialization. The medical devices of the present invention include coatings for controlling the elution rates of the drugs.

### Discussion of the Related Art

Many individuals suffer from circulatory disease caused by a progressive blockage of the blood vessels that perfuse the heart and other major organs. More severe blockage of blood vessels in such individuals often leads to hypertension, ischemic injury, stroke, or myocardial infarction. Atherosclerotic lesions, which limit or obstruct coronary blood flow, are the major cause of ischemic heart disease. Percutaneous transluminal coronary angioplasty is a medical procedure whose purpose is to increase blood flow through an artery. Percutaneous transluminal coronary angioplasty is the predominant treatment for coronary vessel stenosis. The increasing use of this procedure is attributable to its relatively high success rate and its minimal invasiveness compared with coronary bypass surgery. Limitations associated with percutaneous transluminal coronary angioplasty are the abrupt closure of the vessel, which may occur immediately after the procedure, and restenosis, which occurs gradually following the procedure. Additionally, restenosis is a chronic problem in patients who have undergone saphenous vein bypass grafting. The mechanism of acute occlusion appears to involve several factors and may result from vascular recoil with resultant closure of the artery and/or deposition of blood platelets and fibrin along the damaged length of the newly opened blood vessel.

Restenosis after percutaneous transluminal coronary angioplasty is a more gradual process initiated by vascular injury. Multiple processes, including thrombosis, inflammation, growth factor and cytokine release, cell proliferation, cell migration and extracellular matrix synthesis each contribute to the restenotic process.

While the exact mechanism of restenosis is not completely understood, the general aspects of the restenosis process have been identified. In the normal arterial wall, smooth muscle cells proliferate at a low rate, approximately less than 0.1 % per day. Smooth muscle cells in the vessel walls exist in a contractile phenotype characterized by 80 to 90 % of the cell cytoplasmic volume occupied with the contractile apparatus. Endoplasmic reticulum, Golgi and free ribosomes are few and are located in the perinuclear region. Extracellular matrix surrounds the smooth muscle cells and is rich in heparin-like glycosylaminoglycans, which are believed to be responsible for maintaining smooth muscle cells in the contractile phenotypic state (Campbell and Campbell, 1985).

Upon pressure expansion of an intracoronary balloon catheter during angioplasty, smooth muscle cells and endothelial cells within the vessel wall become injured, initiating a thrombotic and inflammatory response. Cell derived growth factors such as platelet derived growth factor, basic fibroblast growth factor, epidermal growth factor, thrombin, etc. released from platelets, invading macrophages and/or leukocytes, or directly from the smooth muscle cells, provoke a proliferative and migratory response in medial smooth muscle cells. These cells undergo a change from the contractile phenotype to a synthetic phenotype characterized by only a few contractile filament bundles, extensive rough endoplasmic reticulum, Golgi and free ribosomes. Proliferation/migration usually begins within 1 to 2 days post-injury and peaks several days thereafter (Campbell and Campbell, 1987; Clowes and Schwartz, 1985).

Daughter cells migrate to the intimal layer of arterial smooth muscle and continue to proliferate and secrete significant amounts of extracellular matrix proteins. Proliferation, migration and extracellular matrix synthesis continue until the damaged endothelial layer is repaired, at which time proliferation slows within the intima, usually within 7 to 14 days post-injury. The newly formed tissue is called neointima. The further vascular narrowing that occurs over the next 3 to 6 months is due primarily to negative or constrictive remodelling.

Simultaneous with local proliferation and migration, inflammatory cells adhere to the site of vascular injury. Within 3 to 7 days post-injury, inflammatory cells have migrated to the deeper layers of the vessel wall. In animal models employing either balloon injury or stent implantation, inflammatory cells may persist at the site of vascular injury for at least 30 days (Tanaka et al., 1993; Edelman et al., 1998). Inflammatory cells therefore are present and may contribute to both the acute and chronic phases of restenosis.

Numerous agents have been examined for presumed anti-proliferative actions in restenosis and have shown some activity in experimental animal models. Some of the agents which have been shown to successfully reduce the extent of intimal hyperplasia in animal models include: heparin and heparin fragments (Clowes, A.W. and Kamovsky M., Nature 265: 25-26, 1977; Guyton, J.R. et al., Circ. Res., 46: 625-634, 1980; Clowes, A.W. and Clowes, M.M., Lab. Invest. 52: 611-616, 1985; Clowes, A.W. and Clowes, M.M., Circ. Res. 58: 839-845, 1986; Majesky et al., Circ. Res. 61: 296-300, 1987; Snow et al., Am. J. Pathol. 137: 313-330, 1990; Okada, T. et al., Neurosurgery 25: 92-98, 1989), colchicine (Currier, J.W. et al., Circ. 80: 11-66, 1989), taxol (Sollot, S.J. et al., J. Clin. Invest. 95: 1869-1876, 1995), angiotensin converting enzyme (ACE) inhibitors (Powell, J.S. et al., Science, 245: 186-188, 1989), angiopeptin (Lundergan, C.F. et al., Am. J. Cardiol. 17( Suppl. B):132B-136B, 1991), cyclosporin A (Jonasson, L. et al., Proc. Natl. Acad. Sci., 85: 2303, 1988), goat-anti-rabbit PDGF antibody (Ferns, G.A.A., et al., Science 253: 1129-1132, 1991), terbinafine (Nemecek, G.M. et al., J. Pharmacol. Exp. Thera. 248: 1167-1174, 1989), trapidil (Liu, M.W. et al., Circ. 81: 1089-1093, 1990), tranilast (Fukuyama, J. et al., Eur. J. Pharmacol. 318: 327-332, 1996), interferon-gamma (Hansson, G.K. and Holm, J., Circ. 84: 1266-1272, 1991), rapamycin (Marx, S.O. et al., Circ. Res. 76: 412-417, 1995), steroids (Colbum, M.D. et al., J. Vasc. Surg. 15: 510-518, 1992), see also Berk, B.C. et al., J. Am. Coll. Cardiol. 17: 111 B-117B, 1991), ionizing radiation (Weinberger, J. et al., Int. J. Rad. Onc. Biol. Phys. 36: 767-775, 1996), fusion toxins (Farb, A. et al., Circ. Res. 80: 542-550, 1997) antisense oligionucleotides (Simons, M. et al., Nature 359: 67-70, 1992) and gene vectors (Chang, M.W. et al., J. Clin. Invest. 96: 2260-2268, 1995). Anti-proliferative action on smooth muscle cells *in vitro* has been demonstrated for many of these agents, including heparin and heparin conjugates, taxol, tranilast, colchicine, ACE inhibitors, fusion toxins, antisense oligionucleotides, rapamycin and ionizing radiation. Thus, agents with diverse mechanisms of smooth muscle cell inhibition may have therapeutic utility in reducing intimal hyperplasia.

However, in contrast to animal models, attempts in human angioplasty patients to prevent restenosis by systemic pharmacologic means have thus far been unsuccessful. Neither aspirin-dipyridamole, ticlopidine, anti-coagulant therapy (acute heparin, chronic warfarin, hirudin or hirulog), thromboxane receptor antagonism nor steroids have been effective in preventing restenosis, although platelet inhibitors have been effective in preventing acute reocclusion after angioplasty (Mak and Topol, 1997; Lang et al., 1991; Popma et al., 1991). The platelet GP IIb/IIIa receptor antagonist, Reopro°is still under study but Reopro°has not shown definitive results for the reduction in restenosis following angioplasty and stenting. Other agents which have also been unsuccessful in the prevention of restenosis include the calcium channel antagonists, prostacyclin mimetics, angiotensin converting enzyme inhibitors, serotonin receptor antagonists and anti-proliferative agents. These agents must be given systemically, however, and attainment of a therapeutically effective dose may not be possible; anti-proliferative (or anti-restenosis) concentrations may exceed the known toxic concentrations of these agents so that levels sufficient to produce smooth muscle inhibition may not be reached (Mak and Topol, 1997; Lang et al., 1991; Popma et al., 1991).

Additional clinical trials in which the effectiveness for preventing restenosis utilizing dietary fish oil supplements or cholesterol lowering agents has been examined, showing either conflicting or negative results so that no pharmacological agents are as yet clinically available to prevent post-angioplasty restenosis (Mak and Topol, 1997; Franklin and Faxon, 1993: Serruys, P.W. et al., 1993). Recent observations suggest that the antilipid/antioxidant agent, probucol, may be useful in preventing restenosis but this work requires confirmation (Tardif et al., 1997; Yokoi, et al., 1997). Probucol is presently not approved for use in the United States and a 30-day pretreatment period would preclude its use in emergency angioplasty. Additionally, the application of ionizing radiation has shown significant promise in reducing or preventing restenosis after angioplasty in patients with stents (Teirstein et al., 1997). Currently, however, the most effective treatments for restenosis are repeat angioplasty, atherectomy or coronary artery bypass grafting, because no therapeutic agents currently have Food and Drug Administration approval for use for the prevention of post-angioplasty restenosis.

Unlike systemic pharmacologic therapy, stents have proven useful in significantly reducing restenosis. Typically, stents are balloon-expandable slotted metal tubes (usually, but not limited to, stainless steel) which, when expanded within the lumen of an angioplastied coronary artery, provide structural support through rigid scaffolding to the arterial wall. This support is helpful in maintaining vessel lumen patency. In two randomized clinical trials, stents increased angiographic success after percutaneous transluminal coronary angioplasty by increasing minimal lumen diameter and reducing, but not eliminating, the incidence of restenosis at 6 months (Serruys et al., 1994; Fischman et al., 1994).

Additionally, the heparin coating of stents appears to have the added benefit of producing a reduction in sub-acute thrombosis after stent implantation (Serruys et al., 1996). Thus, sustained mechanical expansion of a stenosed coronary artery with a stent has been shown to provide some measure of restenosis prevention, and the coating of stents with heparin has demonstrated both the feasibility and the clinical usefulness of delivering drugs locally, at the site of injured tissue.

As stated above, the use of heparin-coated stents demonstrates the feasibility and clinical usefulness of local drug delivery; however, the manner in which the particular drug or drug combination is affixed to the local delivery device will play a role in the efficacy of this type of treatment. For example, the processes and materials utilized to affix the drug/drug combinations to the local delivery device should not interfere with the operations of the drug/drug combinations. In addition, the processes and materials utilized should be biocompatible and maintain the drug/drug combinations on the local device through delivery and over a given period of time. For example, removal of the drug/drug combination during delivery of the local delivery device may potentially cause failure of the device.

Accordingly, there exists a need for drug/drug combinations and associated local delivery devices for the prevention and treatment of vascular injury causing intimal thickening which is either biologically induced, for example, through atherosclerosis, or mechanically induced, for example through percutaneous transluminal coronary angioplasty. In addition, there exists a need for maintaining the drug/drug combinations on the local delivery device through delivery and positioning as well as ensuring that the drug/drug combination is released in therapeutic dosages over a given period of time.

A variety of stent coatings and compositions have been proposed for the prevention and treatment of injury causing intimal thickening. The coatings may be capable themselves of reducing the stimulus the stent provides to the injured lumen wall, thus reducing the tendency towards thrombosis or restenosis. Alternately, the coating may deliver a drug to the lumen that reduces smooth muscle tissue proliferation or restenosis. The mechanism for delivery of the drug is through diffusion of the drug through either a bulk polymer or through pores that are created in the polymer structure, or by erosion of a biodegradable coating.

Both bioabsorbable and biostable compositions have been reported as coatings for stents. They generally have been polymeric coatings that either encapsulate a drug, e.g. rapamycin, taxol etc., or bind such a drug to the surface, e.g. heparin-coated stents. These coatings are applied to the stent in a number of ways, including, though not limited to, dip, spray, or spin coating processes.

One class of biostable materials that has been reported as coatings for stents is polyfluoro homopolymers. Polytetrafluoroethylene (PTFE) homopolymers have been used as implants for many years. These homopolymers are not soluble in any solvent at reasonable temperatures and therefore are difficult to coat onto small medical devices while maintaining important features of the devices (e.g. slots in stents).

Stents with coatings made from polyvinylidenefluoride homopolymers and containing drugs for release have been suggested. However, like most crystalline polyfluoro homopolymers, they are difficult to apply as high quality films onto surfaces without subjecting them to relatively high temperatures that correspond to the melting temperature of the polymer.

It would be advantageous to develop coatings for implantable medical devices that will reduce thrombosis, restenosis or other adverse reactions, that may include, but do not require, the use of drugs to achieve such effects and that possess physical and mechanical properties effective for use in such devices even when such coated devices are subjected to relatively low maximum temperatures. It would also be advantageous to develop implantable medical devices in combination with various drugs which treat disease and minimize or substantially eliminate a living organism's reaction to the implantation of the medical device. In certain circumstances, it may be advantageous to develop implantable medical devices in combination with various drugs which promote wound healing and endothelialization of the medical device.

It would also be advantageous to develop coatings for implantable medical devices that allow for the precise control of the elution rate of drugs from the implantable medical devices.

It would also be advantageous to develop delivery devices that provide for the release of one or more drugs that act through different molecular mechanisms affecting cell proliferation.

Another type of vascular disease of considerable concern is atherosclerosis. Atherosclerosis is a thickening and hardening of the arteries and is generally believed to be caused by the progressive build-up of fatty substances, e.g. cholesterol, inflammatory cells, cellular waste products, calcium and other substances in the inner lining or intima of the arteries. The build-up of these irritating substances may in turn stimulate cells in the walls of the affected arteries to produce additional substances that result in the further build-up of cells leading to the growth of a lesion. This build-up or lesion is generally referred to as plaque.

Recent studies have led to a shift in the understanding of atherosclerosis and uncovered another major vascular problem not yet well treated. Scientists theorize that at least some coronary disease is an inflammatory process in which inflammation causes plaque to destabilize and rupture. This inflamed plaque is known as atherosclerotic vulnerable plaque.

Vulnerable plaque consists of a lipid-rich core covered by a thin layer of smooth muscle cells. These vulnerable plaques are prone to rupture and erosion and can cause significant infarcts if the thin cellular layer ruptures or ulcerates. When the inflammatory cells erode or rupture, the lipid core is exposed to the blood flow, forming thrombi in the artery. These thrombi may grow rapidly and block the artery or detach and travel downstream, leading to embolic events, unstable angina, myocardial infarction and/or sudden death. In fact, some recent studies have suggested that plaque rupture may trigger 60 to 70 % of all fatal myocardial infarctions. See US-A-5,924,997 and US-A-6,245,026 for further descriptions of vulnerable plaques.

Early methods used to detect atherosclerosis lacked the diagnostic tools to visualize and identify vulnerable plaque in cardiac patients. However, new diagnostic technologies are under development to identify the location of vulnerable plaques in the coronary arteries. These new devices include refined magnetic resonance imaging (MRI), thermal sensors that measure the temperature of the arterial wall, on the premise that the inflammatory process generates heat, elasticity sensors, intravascular ultrasound, optical coherence tomography (OCT), contrast agents and near-infrared and infrared light. What is not currently clear, however, is how to treat these vulnerable plaque lesions once they are found.

Treating vulnerable plaque by using balloon angioplasty followed by traditional stenting would provide less than satisfactory results. Balloon angioplasty by itself may rupture the vulnerable plaque, exposing the underlying fresh tissue cells, collagen or damaged endothelium to the blood flow. This condition ultimately leads to the formation of a thrombus or blood clot that may partially or completely occlude the vessel. In addition, while bare or uncoated stents will induce neointimal hyperplasia that will provide a protective cover over the vulnerable plaque, restenosis remains a major problem that may create more risk to the patient than the original vulnerable plaque.

Accordingly, it would be advantageous to develop a drug-eluting stent that effectively treats vulnerable plaque and related vascular disease.

WO 01/97809 discloses a method of treating or inhibiting cardiovascular, cerebral vascular or peripheral vascular disease in a mammal in need thereof which comprises providing said mammal with an effective amount of a rapamycin, which may be provided as part of a combination regime, and may be administered via implants.

EP-A-1 362 602 discloses a medical device for the treatment of vascular disease. The medical device, which may be a stent, comprises a scaffold structure for maintaining luminal patency, a biocompatible vehicle affixed to at least a portion of the scaffold structure, and one or more agents in therapeutic dosages incorporated into the biocompatible vehicle, wherein the biocompatible vehicle is configured to release the one or more agents over one or more time periods to treat both an acute phase and a chronic phase of the vascular disease. One of the agents referred to is rapamycin.

EP-A-1 586 338, which is a reference under Article 54(3) EPC, discloses a drug-eluting medical device, such as a stent, comprising: an implantable intraluminal structure; a pharmaceutically active agent, which may be rapamycin, in therapeutic dosages, affixed to at least a portion of the implantable transluminal structure; and a stabilising agent mixed with the pharmaceutically active agent to substantially hinder degradation thereof through oxidation.

### SUMMARY OF THE INVENTION

The present invention provides a medical device for the treatment of atherosclerotic vulnerable plaque as defined in claim 1. Preferred features of the invention are defined in the dependent claims.

As used herein, rapamycin includes rapamycin and all analogs, derivatives and conjugates that bind to FKBP12, and other immunophilins and possesses the same pharmacologic properties as rapamycin including inhibition of TOR.

The medical devices of the present invention provide a means for overcoming the difficulties associated with the methods and devices currently in use for the treatment of vulnerable plaque and other related vascular disease, as briefly described above.

The medical devices of the present invention utilize a combination of materials to treat disease and reactions by living organisms due to the implantation of medical devices for the treatment of disease or other conditions. The local delivery of the drugs generally substantially reduces the potential toxicity of the drugs when compared to systemic delivery while increasing their efficacy.

The drugs affixed to the medical devices may be used to treat various diseases. The drugs may also be affixed to minimize or substantially eliminate the biological organism's reaction to the introduction of the medical device utilized to treat a separate condition. The stents may be introduced to open coronary arteries or other body lumens such as biliary ducts. The introduction of these stents causes a smooth muscle cell proliferation effect as well as inflammation.

In order to be effective, the drugs should preferably remain on the medical devices during delivery and implantation. Accordingly, various coating techniques for creating strong bonds between the drugs may be utilized. In addition, various materials may be utilized as surface modifications to prevent the drugs from coming off prematurely.

### BRIEF DESCRIPTION OF THE DRAWINGS

The foregoing and other features and advantages of the invention will be apparent from the following, more particular description, as illustrated in the accompanying drawings.
Figure 1 is a view along the length of a stent (ends not shown) prior to expansion showing the exterior surface of the stent and the characteristic banding pattern.
Figure 2 indicates the fraction of drug released as a function of time from coatings over which no topcoat has been disposed.
Figure 3 indicates the fraction of drug released as a function of time from coatings including a topcoat disposed thereon.
Figure 4 indicates the fraction of drug released as a function of time from coatings over which no topcoat has been disposed.
Figure 5 indicates *in vivo* stent release kinetics of rapamycin from poly(VDF/HFP).
Figure 6 is a graphical representation of the anti-proliferative activity of Panzem® in synchronized cultured human coronary artery smooth muscle cells stimulated with 2 % fetal bovine serum.
Figure 7 is a graphical representation of the anti-proliferative activity of rapamycin in synchronized cultured human coronary artery smooth muscle cells stimulated with 2 % fetal bovine serum.
Figure 8 is a graphical representation of the anti-proliferative activity of rapamycin with varying concentrations of Panzem® in synchronized cultured human coronary artery smooth muscle cells stimulated with 2 % fetal bovine serum.
Figure 9 is a graphical representation of a MTS assay of Panzem®.
Figure 10 is a graphical representation of the *in vitro* release kinetics of rapamycin from a layered rapamycin, Panzem® and polymeric coating.
Figure 11 is a graphical representation of the *in vitro* release kinetics of Panzem® from a layered rapamycin, Panzem® and polymeric coating.
Figure 12 indicates the fraction or percentage of rapamycin released over time from various polymeric coatings during *in vivo* testing.
Figure 13 indicates the fraction or percentage of rapamycin released over time from various polymeric coatings during *in vitro* testing.

### DETAILED DESCRIPTION

The medical devices of the present invention may be utilized to effectively prevent and treat vascular disease, and in particular, vascular disease caused by injury. Various medical treatment devices utilized in the treatment of vascular disease may ultimately induce further complications. For example, balloon angioplasty is a procedure utilized to increase blood flow through an artery and is the predominant treatment for coronary vessel stenosis. However, as stated above, the procedure typically causes a certain degree of damage to the vessel wall, thereby potentially exacerbating the problem at a point later in time. Although other procedures and diseases may cause similar injury, exemplary embodiments will be described with respect to the treatment of restenosis and related complications following percutaneous transluminal coronary angioplasty.

As stated previously, the implantation of a coronary stent in conjunction with balloon angioplasty is highly effective in treating acute vessel closure and may reduce the risk of restenosis. Intravascular ultrasound studies (Mintz et al., 1996) suggest that coronary stenting effectively prevents vessel constriction and that most of the late luminal loss after stent implantation is due to plaque growth, probably related to neointimal hyperplasia. The late luminal loss after coronary stenting is almost two times higher than that observed after conventional balloon angioplasty. Thus, inasmuch as stents prevent at least a portion of the restenosis process, a combination of drugs which prevents smooth muscle cell proliferation, reduces inflammation and reduces coagulation or prevents smooth muscle cell proliferation by multiple mechanisms, reduces inflammation and reduces coagulation combined with a stent may provide the most efficacious treatment for post-angioplasty restenosis.

The local delivery of drug combinations from a stent has the following advantages; namely, the prevention of vessel recoil and remodelling through the scaffolding action of the stent and the prevention of multiple components of neointimal hyperplasia or restenosis as well as a reduction in inflammation and thrombosis. This local administration of drugs to stented coronary arteries may also have additional therapeutic benefit. For example, higher tissue concentrations of the drugs may be achieved utilizing local delivery, rather than systemic administration. In addition, reduced systemic toxicity may be achieved utilizing local delivery rather than systemic administration while maintaining higher tissue concentrations. Also, in utilizing local delivery from a stent rather than systemic administration, a single procedure may suffice with better patient compliance. An additional benefit of combination drug therapy may be to reduce the dose of each of the drugs, thereby limiting their toxicity, while still achieving a reduction in restenosis, inflammation and thrombosis. Local stent-based therapy is therefore a means of improving the therapeutic ratio (efficacy/toxicity) of anti-restenosis, anti-inflammatory, anti-thrombotic drugs.

There are a multiplicity of different stents that may be utilized following percutaneous transluminal coronary angioplasty. Although any number of stents may be utilized in accordance with the present invention, for simplicity, a limited number of stents will be described in exemplary embodiments. The skilled artisan will recognize that any number of stents may be utilized in connection with the present invention.

A stent is commonly used as a tubular structure left inside the lumen of a duct to relieve an obstruction. Commonly, stents are inserted into the lumen in a non-expanded form and are then expanded autonomously, or with the aid of a second device in situ. A typical method of expansion occurs through the use of a catheter-mounted angioplasty balloon which is inflated within the stenosed vessel or body passageway in order to shear and disrupt the obstructions associated with the wall components of the vessel and to obtain an enlarged lumen.

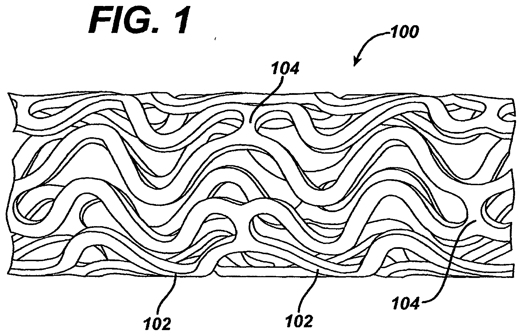

Figure 1 illustrates an exemplary stent 100 which may be utilized in accordance with an exemplary embodiment of the present invention. The expandable cylindrical stent 100 comprises a fenestrated structure for placement in a blood vessel, duct or lumen to hold the vessel, duct or lumen open, more particularly for protecting a segment of artery from restenosis after angioplasty. The stent 100 may be expanded circumferentially and maintained in an expanded configuration that is circumferentially or radially rigid. The stent 100 is axially flexible and when flexed at a band, the stent 100 avoids any externally protruding component parts.

The stent 100 generally comprises first and second ends with an intermediate section therebetween. The stent 100 has a longitudinal axis and comprises a plurality of longitudinally disposed bands 102, wherein each band 102 defines a generally continuous wave along a line segment parallel to the longitudinal axis. A plurality of circumferentially arranged links 104 maintain the bands 102 in a substantially tubular structure. Essentially, each longitudinally disposed band 102 is connected, at a plurality of periodic locations, by a short circumferentially arranged link 104 to an adjacent band 102. The wave associated with each of the bands 102 has approximately the same fundamental spatial frequency in the intermediate section, and the bands 102 are so disposed that the waves associated with them are generally aligned so as to be generally in phase with one another. As illustrated in the figure, each longitudinally arranged band 102 undulates through approximately two cycles before there is a link to an adjacent band 102.

The stent 100 may be fabricated utilizing any number of methods. For example, the stent 100 may be fabricated from a hollow or formed stainless steel tube that may be machined using lasers, electric discharge milling, chemical etching or other means. The stent 100 is inserted into the body and placed at the desired site in an unexpanded form. In one exemplary embodiment, expansion may be effected in a blood vessel by a balloon catheter, where the final diameter of the stent 100 is a function of the diameter of the balloon catheter used.

It should be appreciated that a stent 100 in accordance with the present invention may be embodied in a shape-memory material, including, for example, an appropriate alloy of nickel and titanium or stainless steel. Structures formed from stainless steel may be made self-expanding by configuring the stainless steel in a predetermined manner, for example, by twisting it into a braided configuration. In this embodiment, after the stent 100 has been formed, it may be compressed so as to occupy a space sufficiently small as to permit its insertion in a blood vessel or other tissue by insertion means, wherein the insertion means include a suitable catheter, or flexible rod. On emerging from the catheter, the stent 100 may be configured to expand into the desired configuration where the expansion is automatic or triggered by a change in pressure, temperature or electrical stimulation.

In one reference example the rapamycin is directly incorporated into a polymeric matrix and sprayed onto the outer surface of the stent. The rapamycin elutes from the polymeric matrix over time and enters the surrounding tissue. The rapamycin preferably remains on the stent for at least 3 days up to approximately 6 months, and more preferably between 7 and 30 days.

Any number of non-erodible polymers may be utilized in conjunction with rapamycin. In one exemplary embodiment, the rapamycin may be incorporated into a film-forming polyfluoro copolymer comprising an amount of a first moiety selected from the group consisting of polymerized vinylidenefluoride and polymerized tetrafluoroethylene, and an amount of a second moiety other than the first moiety and which is copolymerized with the first moiety, thereby producing the polyfluoro copolymer, the second moiety being capable of providing toughness or elastomeric properties to the polyfluoro copolymer, wherein the relative amounts of the first moiety and the second moiety are effective to provide the coating and film produced therefrom with properties effective for use in treating implantable medical devices.

The present invention provides implantable medical devices which are stents coated with a film of the polymeric coating in amounts effective to reduce thrombosis and/or restenosis when such stents are used in, for example, angioplasty procedures. As used herein, polyfluoro copolymers means those copolymers comprising an amount of a first moiety selected from the group consisting of polymerized vinylidenefluoride and polymerized tetrafluoroethylene, and an amount of a second moiety other than the first moiety and which is copolymerized with the first moiety to produce the polyfluoro copolymer, the second moiety being capable of providing toughness or elastomeric properties to the polyfluoro copolymer, wherein the relative amounts of the first moiety and the second moiety are effective to provide coatings and film made from such polyfluoro copolymers with properties effective for use in coating implantable medical devices.

The coatings comprise drugs for reducing restenosis, inflammation, and/or thrombosis, and stents coated with such coatings may provide sustained release of the drugs. Films prepared from certain polyfluoro copolymer coatings provide the physical and mechanical properties required of conventional coated medical devices, even where maximum temperature, to which the device coatings and films are exposed, are limited to relatively low temperatures. This is particularly important when using the coating/film to deliver drugs that are heat sensitive, or when applying the coating onto temperature-sensitive devices. When maximum exposure temperature is not an issue, for example, where heat-stable drugs are incorporated into the coatings, higher melting thermoplastic polyfluoro copolymers may be used and, if very high elongation and adhesion is required, elastomers may be used. If desired or required, the polyfluoro elastomers may be crosslinked by standard methods described in, e.g., Modern Fluoropolymers, (J. Shires ed.), John Wiley & Sons, New York, 1997, pp. 77-87.

The polyfluoro copolymers provide improved biocompatible coatings or vehicles for medical devices. These coatings provide inert biocompatible surfaces to be in contact with body tissue of a mammal, for example, a human, sufficient to reduce restenosis, or thrombosis, or other undesirable reactions. While many reported coatings made from polyfluoro homopolymers are insoluble and/or require high heat, for example, greater than about 125°C, to obtain films with adequate physical and mechanical properties for use on stents, or are not particularly tough or elastomeric, films prepared from the polyfluoro copolymers referred to above provide adequate adhesion, toughness or elasticity, and resistance to cracking when formed on medical devices. In certain exemplary embodiments, this is the case even where the devices are subjected to relatively low maximum temperatures.

The polyfluoro copolymers used for such coatings are preferably film-forming polymers that have molecular weight high enough so as not to be waxy or tacky. The polymers and films formed therefrom should preferably adhere to the stent and not be readily deformable after deposition on the stent as to be able to be displaced by hemodynamic stresses. The polymer molecular weight should preferably be high enough to provide sufficient toughness so that films comprising the polymers will not be rubbed off during handling or deployment of the stent. In certain exemplary embodiments the coating will not crack where expansion of the stent occurs.

Coatings may comprise polyfluoro copolymers, as defined hereinabove. The second moiety polymerized with the first moiety to prepare the polyfluoro copolymer may be selected from those polymerized, biocompatible monomers that would provide biocompatible polymers acceptable for implantation in a mammal, while maintaining sufficient elastomeric film properties for use on medical devices claimed herein. Such monomers include, without limitation, hexafluoropropylene (HFP), tetrafluoroethylene (TFE), vinylidenefluoride, 1-hydropentafluoropropylene, perfluoro(methyl vinyl ether), chlorotrifluoroethylene (CTFE), pentafluoropropene, trifluoroethylene, hexafluoroacetone and hexafluoroisobutylene.

Such polyfluoro copolymers typically comprise vinylidinefluoride copolymerized with hexafluoropropylene, in the weight ratio in the range of from about 50 to about 92 weight % vinylidinefluoride to about 50 to about 8 weight % HFP. Preferably, such polyfluoro copolymers comprise from about 50 to about 85 weight % vinylidinefluoride copolymerized with from about 50 to about 15 weight % HFP. More preferably, the polyfluoro copolymers will comprise from about 55 to about 70 weight % vinylidinefluoride copolymerized with from about 45 to about 30 weight % HFP. Even more preferably, polyfluoro copolymers comprise from about 55 to about 65 weight % vinylidinefluoride copolymerized with from about 45 to about 35 weight % HFP. Such polyfluoro copolymers are soluble, in varying degrees, in solvents such as dimethylacetamide (DMAc), tetrahydrofuran, dimethyl formamide, dimethyl sulfoxide and n-methyl pyrrolidone. Some are soluble in methylethylketone (MEK), acetone, methanol and other solvents commonly used in applying coatings to conventional implantable medical devices.

Conventional polyfluoro homopolymers are crystalline and difficult to apply as high quality films onto metal surfaces without exposing the coatings to relatively high temperatures that correspond to the melting temperature (Tm) of the polymer. The elevated temperature serves to provide films prepared from such PVDF homopolymer coatings that exhibit sufficient adhesion of the film to the device, while preferably maintaining sufficient flexibility to resist film cracking upon expansion/contraction of the coated medical device. Certain films and coatings provide these same physical and mechanical properties, or essentially the same properties, even when the maximum temperatures to which the coatings and films are exposed is less than about a maximum predetermined temperature. This is particularly important when the coatings/films comprise drugs that are heat sensitive, for example, subject to chemical or physical degradation or other heat-induced negative effects, or when coating heat sensitive substrates of medical devices, for example, subject to heat-induced compositional or structural degradation.

Depending on the particular device upon which the coatings and films are to be applied and the particular use/result required of the device, polyfluoro copolymers used to prepare such devices may be crystalline, semi-crystalline or amorphous.

Where devices have no restrictions or limitations with respect to exposure of same to elevated temperatures, crystalline polyfluoro copolymers may be employed. Crystalline polyfluoro copolymers tend to resist the tendency to flow under applied stress or gravity when exposed to temperatures above their glass transition (Tg) temperatures. Crystalline polyfluoro copolymers provide tougher coatings and films than their fully amorphous counterparts. In addition, crystalline polymers are more lubricious and more easily handled through crimping and transfer processes used to mount self-expanding stents, for example, nitinol stents.

Semi-crystalline and amorphous polyfluoro copolymers are advantageous where exposure to elevated temperatures is an issue, for example, where heat-sensitive drugs are incorporated into the coatings and films, or where device design, structure and/or use preclude exposure to such elevated temperatures. Semi-crystalline polyfluoro copolymer elastomers comprising relatively high levels, for example, from about 30 to about 45 weight % of the second moiety, for example, HFP, copolymerized with the first moiety, for example, VDF, have the advantage of reduced coefficient of friction and self-blocking relative to amorphous polyfluoro copolymer elastomers. Such characteristics may be of significant value when processing, packaging and delivering medical devices coated with such polyfluoro copolymers. In addition, such polyfluoro copolymer elastomers comprising such relatively high content of the second moiety serves to control the solubility of certain drugs, for example, rapamycin, in the polymer and therefore controls permeability of the drug through the matrix.

Polyfluoro copolymers may be prepared by various known polymerization methods. For example, high pressure, free-radical, semi-continuous emulsion polymerization techniques such as those disclosed in Fluoroelastomers-dependence of relaxation phenomena on compositions, POLYMER 30, 2180, 1989, by Ajroldi, et al., may be employed to prepare amorphous polyfluoro copolymers, some of which may be elastomers. In addition, free-radical batch emulsion polymerization techniques disclosed herein may be used to obtain polymers that are semi-crystalline, even where relatively high levels of the second moiety are included.

As described above, stents may comprise a wide variety of materials and a wide variety of geometries. Stents may be made of biocomptible materials, including biostable and bioabsorbable materials. Suitable biocompatible metals include, but are not limited to, stainless steel, tantalum, titanium alloys (including nitinol), and cobalt alloys (including cobalt-chromium nickel alloys). Suitable nonmetallic biocompatible materials include, but are not limited to, polyamides, polyolefins (i.e. polypropylene, polyethylene etc.), nonabsorbable polyesters (i.e. polyethylene terephthalate), and bioabsorbable aliphatic polyesters (i.e. homopolymers and copolymers of lactic acid, glycolic acid, lactide, glycolide, para-dioxanone, trimethylene carbonate, epsilon - caprolactone, and blends thereof).

The film-forming biocompatible polymer coatings generally are applied to the stent in order to reduce local turbulence in blood flow through the stent, as well as adverse tissue reactions. The coatings and films formed therefrom are also used to administer drugs to the site of the stent placement. Generally, the amount of polymer coating to be applied to the stent will vary depending on, among other possible parameters, the particular polyfluoro copolymer used to prepare the coating, the stent design and the desired effect of the coating. Generally, the coated stent will comprise from about 0.1 to about 15 weight % of the coating, preferably from about 0.4 to about 10 weight %. The polyfluoro copolymer coatings may be applied in one or more coating steps, depending on the amount of polyfluoro copolymer to be applied. Different polyfluoro copolymers may be used for different layers in the stent coating. In fact, in certain exemplary embodiments, it is highly advantageous to use a diluted first coating solution comprising a polyfluoro copolymer as a primer to promote adhesion of a subsequent polyfluoro copolymer coating layer that may include drugs. The individual coatings may be prepared from different polyfluoro copolymers.

Additionally, a top coating may be applied to delay release of the drug, or they could be used as the matrix for the delivery of a different drug. Layering of coatings may be used to stage release of the drugs or to control release of different drugs placed in different layers.

Blends of polyfluoro copolymers may also be used to control the release rate of different agents or to provide a desirable balance of coating properties, i.e. elasticity, toughness, etc., and drug delivery characteristics, for example, release profile. Polyfluoro copolymers with different solubilities in solvents may be used to build up different polymer layers that may be used to deliver different drugs or to control the release profile of a drug. For example, polyfluoro copolymers comprising 85.5/14.5 (wt/wt) of poly(vinylidinefluoride/HFP) and 60.6/39.4 (wt/wt) of poly(vinylidinefluoride /HFP) are both soluble in DMAc. However, only the 60.6/39.4 PVDF polyfluoro copolymer is soluble in methanol. So, a first layer of the 85.5/14.5 PVDF polyfluoro copolymer comprising a drug could be over coated with a topcoat of the 60.6/39.4 PVDF polyfluoro copolymer made with the methanol solvent. The top coating may be used to delay the drug delivery of the drug contained in the first layer. Alternately, the second layer could comprise a different drug to provide for sequential drug delivery. Multiple layers of different drugs could be provided by alternating layers of first one polyfluoro copolymer, then the other. As will be readily appreciated by those skilled in the art, numerous layering approaches may be used to provide the desired drug delivery.

Coatings may be formulated by mixing one or more drugs with the coating polyfluoro copolymers in a coating mixture. The drug may be present as a liquid, a finely divided solid, or any other appropriate physical form. Optionally, the coating mixture may include one or more additives, for example, nontoxic auxiliary substances such as diluents, carriers, excipients, stabilizers or the like. Other suitable additives may be formulated with the polymer and drug. For example, a hydrophilic polymer may be added to a biocompatible hydrophobic coating to modify the release profile, or a hydrophobic polymer may be added to a hydrophilic coating to modify the release profile. One example would be adding a hydrophilic polymer selected from the group consisting of polyethylene oxide, polyvinyl pyrrolidone, polyethylene glycol, carboxylmethyl cellulose, and hydroxymethyl cellulose to a polyfluoro copolymer coating to modify the release profile. Appropriate relative amounts may be determined by monitoring the *in vitro* and/or *in vivo* release profiles for the drugs.

The best conditions for the coating application are when the polyfluoro copolymer and drug have a common solvent. This provides a wet coating that is a true solution. Less desirable, yet still usable, are coatings that contain the drug as a solid dispersion in a solution of the polymer in solvent. Under the dispersion conditions, care must be taken to ensure that the particle size of the dispersed pharmaceutical powder, both the primary powder size and its aggregates and agglomerates, is small enough not to cause an irregular coating surface or to clog the slots of the stent that need to remain essentially free of coating. In cases where a dispersion is applied to the stent and the smoothness of the coating film surface requires improvement, or to be ensured that all particles of the drug are fully encapsulated in the polymer, or in cases where the release rate of the drug is to be slowed, a clear (polyfluoro copolymer only) topcoat of the same polyfluoro copolymer used to provide sustained release of the drug or another polyfluoro copolymer that further restricts the diffusion of the drug out of the coating may be applied. The topcoat may be applied by dip coating with mandrel to clear the slots. This method is disclosed in US-A-6 153 252. Other methods for applying the topcoat include spin coating and spray coating. Dip coating of the topcoat can be problematic if the drug is very soluble in the coating solvent, which swells the polyfluoro copolymer, and the clear coating solution acts as a zero concentration sink and redissolves previously deposited drug. The time spent in the dip bath may need to be limited so that the drug is not extracted out into the drug-free bath. Drying should be rapid so that the previously deposited drug does not completely diffuse into the topcoat.

The amount of drug will be dependent upon the particular drug employed and medical condition being treated. Typically, the amount of drug represents about 0.001 % to about 70 % of the total coating weight, more typically about 0.001 % to about 60 % of the total coating weight. It is possible that the drug may represent as little as 0.0001 % of the total coating weight.

The quantity and type of polyfluoro copolymers employed in the coating film comprising the drug will vary depending on the release profile desired and the amount of drug employed. The product may contain blends of the same or different polyfluoro copolymers having different molecular weights to provide the desired release profile or consistency to a given formulation.

Polyfluoro copolymers may release dispersed drug by diffusion. This can result in prolonged delivery (over, say approximately 1 to 2,000 hours, preferably 2 to 800 hours) of effective amounts (0.001 µg/cm²-min to 1,000 µg/cm²-min) of the drug. The dosage may be tailored to the subject being treated, the severity of the affliction, the judgment of the prescribing physician, and the like.

Individual formulations of drugs and polyfluoro copolymers may be tested in appropriate *in vitro* and *in vivo* models to achieve the desired drug release profiles. For example, a drug could be formulated with a polyfluoro copolymer or blend of polyfluoro copolymers, coated onto a stent and placed in an agitated or circulating fluid system, for example, 25 % ethanol in water. Samples of the circulating fluid could be taken to determine the release profile (such as by HPLC, UV analysis or use of radiotagged molecules). The release of a drug from a stent coating into the interior wall of a lumen could be modelled in an appropriate animal system. The drug release profile could then be monitored by appropriate means, such as by taking samples at specific times and assaying the samples for drug concentration (using HPLC to detect drug concentration). Thrombus formation can be modelled in animal models using the In-platelet imaging methods described by Hanson and Harker, Proc. Natl. Acad. Sci. USA 85:3184-3188 (1988). Following this or similar procedures, those skilled in the art will be able to formulate a variety of stent coating formulations.

While not a requirement of the present invention, the coatings and films may be crosslinked once applied to the medical devices. Crosslinking may be effected by any of the known crosslinking mechanisms, such as chemical, heat or light. In addition, crosslinking initiators and promoters may be used where applicable and appropriate. In those exemplary embodiments utilizing crosslinked films comprising drugs, curing may affect the rate at which the drug diffuses from the coating.

### EXAMPLES

### Example for reference 1:

A PVDF homopolymer (Solef® 1008 from Solvay Advanced Polymers, Houston, TX,°about 175°C) and polyfluoro copolymers of poly(vinylidenefluoride/HFP), 92/8 and 91/9 weight % vinylidenefluoride/HFP as determined by F¹⁹ NMR, respectively (eg: Solef® 11010 and 11008, Solvay Advanced Polymers, Houston, TX,°about 159°C and 160°C, respectively) were examined as potential coatings for stents. These polymers are soluble in solvents such as, but not limited to, DMAc, N,N-dimethylformamide (DMF), dimethyl sulfoxide (DMSO), N-methylpyrrolidone (NMP), tetrahydrofuran (THF) and acetone. Polymer coatings were prepared by dissolving the polymers in acetone, at 5 weight % as a primer, or by dissolving the polymer in 50/50 DMAc/acetone, at 30 weight % as a topcoat. Coatings that were applied to the stents by dipping and dried at 60°C in air for several hours, followed by 60°C for 3 hours in a <100 mm Hg vacuum, resulted in white foamy films. As applied, these films adhered poorly to the stent and flaked off, indicating they were too brittle. When stents coated in this manner were heated above 175°C, i.e. above the melting temperature of the polymer, a clear, adherent film was formed. Such coatings require high temperatures, for example, above the melting temperature of the polymer, to achieve high quality films. As mentioned above, the high temperature heat treatment is unacceptable for the majority of drugs due to their thermal sensitivity.

### Example for reference 2:

A polyfluoro copolymer (Solef® 21508) comprising 85.5 weight % vinylidenefluoride copolymerized with 14.5 weight % HFP, as determined by F¹⁹ NMR, was evaluated. This copolymer is less crystalline than the polyfluoro homopolymer and copolymers described in Example 1. It also has a lower melting point reported to be about 133°C. Once again, a coating comprising about 20 weight % of the polyfluoro copolymer was applied from a polymer solution in 50/50 DMAc/MEK. After drying (in air) at 60°C for several hours, followed by 60°C for 3 hours in a <100 mtorr Hg vacuum, clear adherent films were obtained. This eliminated the need for a high temperature heat treatment to achieve high quality films. Coatings were smoother and more adherent than those of Example 1. Some coated stents that underwent expansion show some degree of adhesion loss and "tenting" as the film pulls away from the metal. Where necessary, modification of coatings containing such copolymers may be made, e.g. by addition of plasticizers or the like to the coating compositions. Films prepared from such coatings may be used to coat stents or other medical devices, particularly where those devices are not susceptible to expansion to the degree of the stents.

The coating process above was repeated, this time with a coating comprising the 85.5/14.6 (wt/wt) (vinylidenefluoride/HFP) and about 30 weight % of rapamycin (Wyeth-Ayerst Laboratories, Philadelphia, PA), based on total weight of coating solids. Clear films that would occasionally crack or peel upon expansion of the coated stents resulted. It is believed that inclusion of plasticizers and the like in the coating composition will result in coatings and films for use on stents that are not susceptible to such cracking and peeling.

### Example for reference 3:

Polyfluoro copolymers of still higher HFP content were then examined. This series of polymers were not semi-crystalline, but rather are marketed as elastomers. One such copolymer is Fluorel® FC2261 Q (from Dyneon, a 3M-Hoechst Enterprise, Oakdale, MN), a 60.6/39.4 (wt/wt) copolymer of vinylidenefluoride/HFP. Although this copolymer has a Tg well below room temperature (Tg about -20°C) it is not tacky at room temperature or even at 60°C. This polymer has no detectable crystallinity when measured by Differential Scanning Calorimetry (DSC) or by wide angle X-ray diffraction. Films formed on stents as described above were non-tacky, clear and expanded without incident when the stents were expanded.

The coating process above was repeated, this time with coatings comprising the 60.6/39.4 (wt/wt) (vinylidenefluoride/HFP) and about 9, 30 and 50 weight % of rapamycin (Wyeth-Ayerst Laboratories, Philadelphia, PA), based on total weight of coating solids, respectively. Coatings comprising about 9 and 30 weight % rapamycin provided white, adherent, tough films that expanded without incident on the stent. Inclusion of 50 % drug, in the same manner, resulted in some loss of adhesion upon expansion.

Changes in the comonomer composition of the polyfluoro copolymer also can affect the nature of the solid state coating, once dried. For example, the semi-crystalline copolymer, Solef® 21508, containing 85.5 % vinylidenefluoride polymerized with 14.5 % by weight HFP forms homogeneous solutions with about 30 % rapamycin (drug weight divided by total solids weight, for example, drug plus copolymer) in DMAc and 50/50 DMAc/MEK. When the film is dried (60°C/16 hours followed by 60°C/3 hours in vacuum of 100 mm Hg) a clear coating, indicating a solid solution of the drug in the polymer, is obtained. Conversely, when an amorphous copolymer, Fluorel^{™} FC2261 Q, of PDVF/HFP at 60.6/39.5 (wt/wt) forms a similar 30 % solution of rapamycin in DMAc/MEK and is similarly dried, a white film, indicating phase separation of the drug and the polymer, is obtained. This second drug-containing film is much slower to release the drug into an *in vitro* test solution of 25 % ethanol in water than is the former clear film of crystalline Solef® 21508. X-ray analysis of both films indicates that the drug is present in a non-crystalline form. Poor or very low solubility of the drug in the high HFP-containing copolymer results in slow permeation of the drug through the thin coating film. Permeability is the product of diffusion rate of the diffusing species (in this case the drug) through the film (the copolymer) and the solubility of the drug in the film.

### Example for reference

### 4: In vitro release results of rapamycin from coating.

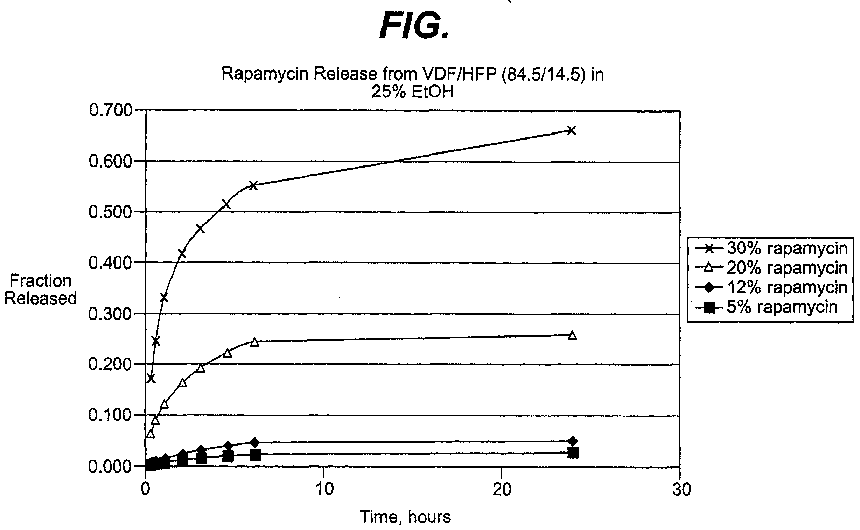

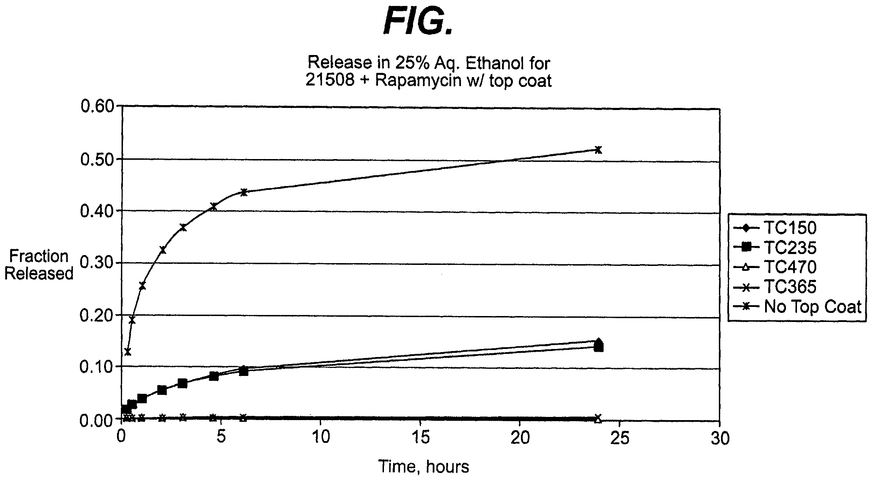

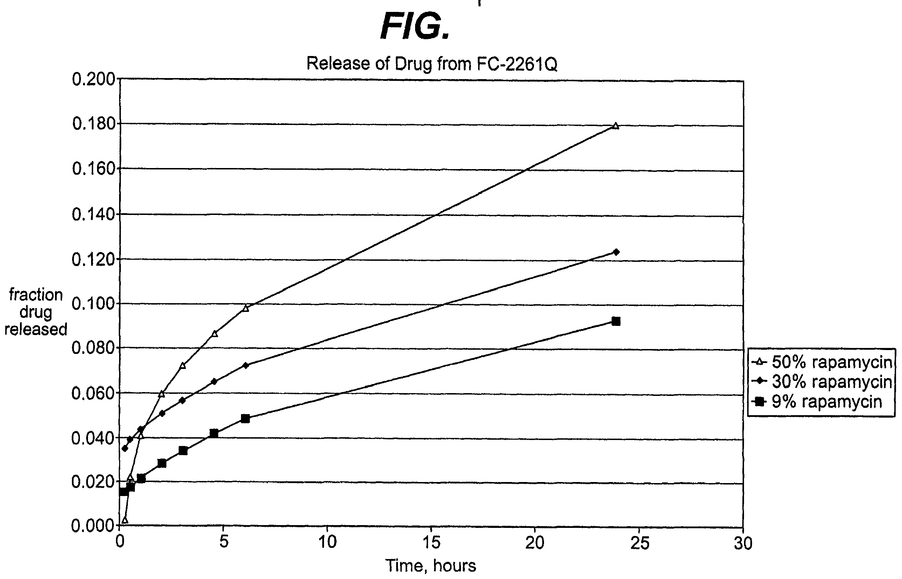

Figure 2 is a plot of data for the 85.5/14.5 vinylidenefluoride/HFP polyfluoro copolymer, indicating fraction of drug released as a function of time, with no topcoat. Figure 3 is a plot of data for the same polyfluoro copolymer over which a topcoat has been disposed, indicating that most effect on release rate is with a clear topcoat. As shown therein, TC150 refers to a device comprising 150 µg of topcoat, TC235 refers to 235 µg of topcoat, etc. The stents before topcoating had an average of 750 µg of coating containing 30 % rapamycin. Figure 4 is a plot for the 60.6/39.4 vinylidenefluoride/HFP polyfluoro copolymer, indicating fraction of drug released as a function of time, showing significant control of release rate from the coating without the use of a topcoat. Release is controlled by loading of drug in the film.

### Example for reference

### 5: In vivo stent release kinetics of rapamycin from poly(VDF/HFP).

9 New Zealand white rabbits (2.5-3.0 kg) on a normal diet were given aspirin 24 hours prior to surgery, again just prior to surgery and for the remainder of the study. At the time of surgery, animals were pre-medicated with Acepromazine (0.1-0.2 mg/kg) and anesthetized with a Ketamine/Xylazine mixture (40 mg/kg and 5 mg/kg, respectively). Animals were given a single intraprocedural dose of heparin (150 IU/kg, i.v.).

Arteriectomy of the right common carotid artery was performed and a 5 F catheter introducer (Cordis, Inc.) placed in the vessel and anchored with ligatures. Iodine contrast agent was injected to visualize the right common carotid artery, brachocephalic trunk and aortic arch. A steerable guide wire (0.036 cm (0.014 inch)/180 cm, Cordis, Inc.) was inserted via the introducer and advanced sequentially into each iliac artery to a location where the artery possesses a diameter closest to 2 mm using the angiographic mapping done previously. Two stents coated with a film made of poly(VDF/HFP):(60.6/39.4) with 30 % rapamycin were deployed in each animal where feasible, one in each iliac artery, using 3.0 mm balloon and inflation to 8-10 ATM for 30 seconds followed after a 1 minute interval by a second inflation to 8-10 ATM for 30 seconds. Follow-up angiographs visualizing both iliac arteries are obtained to confirm correct deployment position of the stent.

At the end of procedure, the carotid artery was ligated and the skin is closed with 3/0 vicryl suture using a one layered interrupted closure. Animals were given butoropanol (0.4 mg/kg, s.c.) and gentamycin (4 mg/kg, i.m.). Following recovery, the animals were returned to their cages and allowed free access to food and water.

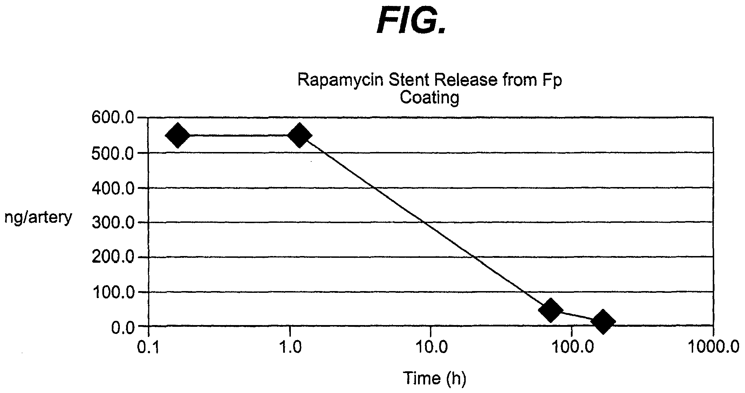

Due to early deaths and surgical difficulties, 2 animals were not used in this analysis. Stented vessels were removed from the remaining 7 animals at the following time points: 1 vessel (1 animal) at 10 minutes post implant; 6 vessels (3 animals) between 40 minutes and 2 hours post-implant (average, 1.2 hours); 2 vessels (2 animals) at 3 days post implant; and 2 vessels (1 animal) at 7 days post-implant. In 1 animal at 2 hours, the stent was retrieved from the aorta rather than the iliac artery. Upon removal, arteries were carefully trimmed at both the proximal and distal ends of the stent. Vessels were then carefully dissected free of the stent, flushed to remove any residual blood, and both stent and vessel frozen immediately, wrapped separately in foil, labelled and kept frozen at -80°C. When all samples had been collected, vessels and stents were frozen, transported and subsequently analyzed for rapamycin in tissue and results are illustrated in Figure 5.

### Example for reference 6: Purifying the polymer.

The Fluorel® FC2261 Q copolymer was dissolved in MEK at about 10 weight % and was washed in a 50/50 mixture of ethanol/water at a 14:1 of ethanol/water to MEK solution ratio. The polymer precipitated out and was separated from the solvent phase by centrifugation. The polymer again was dissolved in MEK and the washing procedure repeated. The polymer was dried after each washing step at 60°C in a vacuum oven (<200 mtorr) over night.

### Example for reference

### 7: In vivo testing of coated stents in porcine coronary arteries.

CrossFlex® stents (available from Cordis, a Johnson & Johnson Company) were coated with the "as received" Fluorel® FC2261 Q PVDF copolymer and with the purified polyfluoro copolymer of Example 6, using the dip and wipe approach. The coated stents were sterilized using ethylene oxide and a standard cycle. The coated stents and bare metal stents (controls) were implanted in porcine coronary arteries, where they remained for 28 days.

Angiography was performed on the pigs at implantation and at 28 days. Angiography indicated that the control uncoated stent exhibited about 21 % restenosis. The polyfluoro copolymer "as received" exhibited about 26 % restenosis(equivalent to the control) and the washed copolymer exhibited about 12.5 % restenosis.

Histology results reported neointimal area at 28 days to be 2.89±0.2, 3.57±0.4 and 2.75±0.3, respectively, for the bare metal control, the unpurified copolymer and the purified copolymer.

Since rapamycin acts by entering the surrounding tissue, it is preferably only affixed to the surface of the stent making contact with one tissue. Typically, only the outer surface of the stent makes contact with the tissue. Accordingly, in one exemplary disclosure only the outer surface of the stent is coated with rapamycin.

It is important to note that the stent, as described above, may be formed from any number of materials, including various metals, polymeric materials and ceramic materials. Accordingly, various technologies may be utilized to immobilize the drug combinations thereon. Specifically, in addition to the polymeric matricies described above biopolymers may be utilized. Biopolymers may be generally classified as natural polymers, while the above-described polymers may be described as synthetic polymers. Exemplary biopolymers, which may be utilized include, agarose, alginate, gelatin, collagen and elastin.

Rapamycin, as described above, is a macroyclic triene antibiotic produced by streptomyces hygroscopicus as disclosed in U.S. Patent No. 3,929,992. It has been found that rapamycin among other things inhibits the proliferation of vascular smooth muscle cells *in vivo.* Accordingly, rapamycin may be utilized in treating intimal smooth muscle cell hyperplasia, restenosis and vascular occlusion in a mammal, particularly following either biologically or mechanically mediated vascular injury, or under conditions that would predispose a mammal to suffering such a vascular injury. Rapamycin functions to inhibit smooth muscle cell proliferation and does not interfere with the re-endothelialization of the vessel walls.

Rapamycin functions to inhibit smooth muscle cell proliferation through a number of mechanisms. In addition, rapamycin reduces the other effects caused by vascular injury, for example, inflammation. The mechanisms of action and various functions of rapamycin are described in detail below.

Rapamycin reduces vascular hyperplasia by antagonizing smooth muscle proliferation in response to mitogenic signals that are released during angioplasty. Inhibition of growth factor and cytokine mediated smooth muscle proliferation at the late G1 phase of the cell cycle is believed to be the dominant mechanism of action of rapamycin. However, rapamycin is also known to prevent T-cell proliferation and differentiation when administered systemically. This is the basis for its immunosuppressive activity and its ability to prevent graft rejection.

The molecular events that are responsible for the actions of rapamycin, a known anti-proliferative, which acts to reduce the magnitude and duration of neointimal hyperplasia, are still being elucidated. It is known, however, that rapamycin enters cells and binds to a high-affinity cytosolic protein called FKBP12. The complex of rapamycin and FKPB12 in turn binds to and inhibits a phosphoinositide (PI)-3 kinase called the "mammalian Target of Rapamycin" or TOR. TOR is a protein kinase that plays a key role in mediating the downstream signaling events associated with mitogenic growth factors and cytokines in smooth muscle cells and T lymphocytes. These events include phosphorylation of p27, phosphorylation of p70 s6 kinase and phosphorylation of 4BP-1, an important regulator of protein translation.

It is recognized that rapamycin reduces restenosis by inhibiting neointimal hyperplasia. However, there is evidence that rapamycin may also inhibit the other major component of restenosis, namely, negative remodelling. Remodelling is a process whose mechanism is not clearly understood but which results in shrinkage of the external elastic lamina and reduction in lumenal area over time, generally a period of approximately 3 to 6 months in humans.

Negative or constrictive vascular remodelling may be quantified angiographically as the % diameter stenosis at the lesion site where there is no stent to obstruct the process. If late lumen loss is abolished in-lesion, it may be inferred that negative remodelling has been inhibited. Another method of determining the degree of remodelling involves measuring in-lesion external elastic lamina area using intravascular ultrasound (IVUS). Intravascular ultrasound is a technique that can image the external elastic lamina as well as the vascular lumen. Changes in the external elastic lamina proximal and distal to the stent from the post-procedural timepoint to 4 month and 12 month follow-ups are reflective of remodelling changes.

Evidence that rapamycin exerts an effect on remodelling comes from human implant studies with rapamycin coated stents showing a very low degree of restenosis in-lesion as well as in-stent. In-lesion parameters are usually measured approximately 5 mm on either side of the stent, i.e. proximal and distal. Since the stent is not present to control remodelling in these zones which are still affected by balloon expansion, it may be inferred that rapamycin is preventing vascular remodelling.

The data in Table 1 below illustrate that in-lesion % diameter stenosis remains low in the rapamycin treated groups, even at 12 months. Accordingly, these results support the hypothesis that rapamycin reduces remodelling.
Angiographic *In-Lesion* % Diameter Stenosis (%, mean ± SD and "n=") In Patients Who Received a Rapamycin-Coated Stent

**TABLE 1.0**

| **Coating Group** | **Post Placement** | **4 - 6 month Follow Up** | **12 month Follow Up** |
|---|---|---|---|
| Brazil | 10.6 ± 5.7 (30) | 13.6 ± 8.6 (30) | 22.3 ± 7.2 (15) |
| Netherlands | 14.7 ± 8.8 | 22.4 ± 6.4 | |

Additional evidence supporting a reduction in negative remodelling with rapamycin comes from intravascular ultrasound data that was obtained from a first-in-man clinical program as illustrated in Table 2 below.

### Matched IVUS data in Patients Who Received a Rapamycin-Coated Stent

**TABLE 2.0**

| **IVUS Parameter** | **Post (n=)** | **4-Month Follow-Up (n=)** | **12-Month Follow-Up (n=)** |
|---|---|---|---|
| Mean proximal vessel area (mm²) | 16.53 ± 3.53 (27) | 16.31 ± 4.36 (28) | 13.96 ± 2.26 (13) |
| Mean distal vessel area (mm²) | 13.12 ± 3.68 (26) | 13.53 ± 4.17 (26) | 12.49 ± 3.25 (14) |

The data illustrated that there is minimal loss of vessel area proximally or distally which indicates that inhibition of negative remodelling has occurred in vessels treated with rapamycin-coated stents.

Other than the stent itself, there have been no effective solutions to the problem of vascular remodelling. Accordingly, rapamycin may represent a biological approach to controlling the vascular remodelling phenomenon.

It may be hypothesized that rapamycin acts to reduce negative remodelling in several ways. By specifically blocking the proliferation of fibroblasts in the vascular wall in response to injury, rapamycin may reduce the formation of vascular scar tissue. Rapamycin may also affect the translation of key proteins involved in collagen formation or metabolism. Rapamycin may be delivered by a coated stent to control negative remodelling of an arterial segment after balloon angioplasty as a means of reducing or preventing restenosis.

Data generated in porcine and rabbit models show that the release of rapamycin into the vascular wall from a non-erodible polymeric stent coating in a range of doses (35-430 µg/15-18 mm coronary stent) produces a peak 50 to 55 % reduction in neointimal hyperplasia as set forth in Table 3 below. This reduction, which is maximal at about 28 to 30 days, is typically not sustained in the range of 90 to 180 days in the porcine model as set forth in Table 4 below.

### Animal Studies with Rapamycin-coated stents. Values are mean ± Standard error of Mean

**TABLE 3.0**

| *Study* | *Duration* | *Stent¹* | *Rapamycin* | *N* | *Neointimal Area (mm²)* | % *Change From* | |
|---|---|---|---|---|---|---|---|
| | | | | | | *Poly mer* | *Metal* |
| ***Porcine*** | | | | | | | |
| 98009 | 14 days | Metal | | 8 | 2.04 ± 0.17 | | |
| | | | | | | | |
| | | 1X + rapamycin | 153 µg | 8 | 1.66 ± 0.17* | -42% | -19% |
| | | 1X + TC300 + rapamycin | 155 µg | | 1.51 ± 0.19* | -47% | -26% |
| | | | | | | | |
| 99005 | 28 days | Metal | | 10 | 2.29 ± 0.21 | | |
| | | | | 9 | 3.91 ± 0.60** | | |
| | | 1X + TC30 + rapamycin | 130 µg | 8 | 2.81 ± 0.34 | | +23% |
| | | 1X + TC100 + rapamycin | 120 µg | 9 | 2.62 ± 0.21 | | +14% |
| | | | | | | | |
| 99006 | 28 days | Metal | | 12 | 4.57 ± 0.46 | | |
| | | EVA/BMA 3X | | 12 | 5.02 ± 0.62 | | +10% |
| | | 1X + rapamycin | 125 µg | 11 | 2.84 ± 0.31*** | -43% | -38% |
| | | 3X + rapamycin | 430 µg | 12 | 3.06 ± 0.17*** | -39% | -33% |
| | | 3X + rapamycin | 157 µg | 12 | 2.77 ± 0.41*** | -45% | -39% |
| | | | | | | | |
| 99011 | 28 days | Metal | | 11 | 3.09 ± 0.27 | | |
| | | | | 11 | 4.52 ± 0.37 | | |
| | | 1X + rapamycin | 189 µg | 14 | 3.05 ± 0.35 | | -1% |
| | | 3X + rapamycin | 182/ 363 µg | 14 | 2.72 ± 0.71 | | -12% |
| | | | | | | | |
| 99021 | 60 days | Metal | | 12 | 2.14 ± 0.25 | | |
| | | 1X + rapamycin | 181 µg | 12 | 2.95 ± 0.38 | | |
| | | | | | | | |
| 99034 | 28 days | Metal | | 8 | 5.24 ± 0.58 | | |
| | | 1X + rapamycin | 186 µg | 8 | 2.47 ± 0.33** | | -53% |
| | | 3X + rapamycin/dex | 185/ 369 µg | 6 | 2.42 ± 0.64** | | -54% |
| 20001 | 28 days | Metal | | 6 | 1.81 ± 0.09 | | |
| | | 1X + rapamycin | 172 µg | 5 | 1.66 ± 0.44 | | -8% |
| | | | | | | | |
| 20007 | 30 days | Metal | | 9 | 2.94 ± 0.43 | | |
| | | 1XTC + rapamycin | 155 µg | 10 | 1.40 ± 0.11* | | -52% |
| | | | | | | | |

| ***Rabbit*** | | | | | | | |
|---|---|---|---|---|---|---|---|
| 99019 | 28 days | Metal | | 8 | 1.20 ± 0.07 | | |
| | | EVA/BMA 1X | | 10 | 1.26 ± 0.16 | | +5% |
| | | 1X + rapamycin | 64 µg | 9 | 0.92 ± 0.14 | -27% | -23% |
| | | 1X + rapamycin | 196 µg | 10 | 0.66 ± 0.12* ** | -48% | -45% |
| | | | | | | | |
| 99020 | 28 days | Metal | | 12 | 1.18 ± 0.10 | | |
| | | EVA/BMA 1X + rapamycin | 197 µg | 8 | 0.81 ± 0.16 | | -32% |

| | | | | | | | |
|---|---|---|---|---|---|---|---|
| ¹Stent nomenclature: EVA/BMA 1X, 2X and 3X signifies approx. 500µg, 1000µg and 1500µg total mass (polymer + drug), respectively. TC: top coat of 30µg, 100 µg or 300µg drug-free BMA; Biphasic: 2 x 1X layers of rapamycin in EVA/BMA separated by a 100µg drug-free BMA layer. 20.05mg/kg/d x 14 d preceded by a loading dose of 0.5mg/kg/d x 3d prior to stent implantation. *p<0.05 from EVA/BMA control. **p<0.05 from Metal; #Inflammation score: (0 = essentially no intimal involvement; 1 = <25% intima involved; 2 = ≥25% intima involved; 3 = >50% intima involved). | | | | | | | |

**TABLE 4.0**

| Study | Duration | Stent¹ | Rapamycin | N | Neointimal Area (mm²) | % Change From | | Inflammation Score # |
|---|---|---|---|---|---|---|---|---|
| | | | | | | Poly mer | Metal | |
| 20007 | 3 days | Metal | | 10 | 0.38 ± 0.06 | | | 1.05 ± 0.06 |
| ETP-2-002233-P | | 1XTC + rapamycin | 155 µg | 10 | 0.29 ± 0.03 | | -24% | 1.08 ± 0.04 |
| | | | | | | | | |
| | 30 days | Metal | | 9 | 2.94 ± 0.43 | | | 0.11 ± 0.08 |
| | | 1XTC + rapamycin | 155 µg | 10 | 1.40 ± 0.11* | | -52% | 0.25 ± 0.10 |
| | | | | | | | | |
| | 90 days | Metal | | 10 | 3.45 ± 0.34 | | | 0.20 ± 0.08 |
| | | 1XTC + rapamycin | 155 µg | 10 | 3.03 ± 0.29 | | -12% | 0.80 ± 0.23 |
| | | 1X + rapamycin | 171 µg | 10 | 2.86 ± 0.35 | | -17% | 0.60 ± 0.23 |
| | | | | | | | | |
| | 180 days | Metal | | 10 | 3.65 ± 0.39 | | | 0.65 ± 0.21 |
| | | 1XTC + rapamycin | 155 µg | 10 | 3.34 ± 0.31 | | -8% | 1.50 ± 0.34 |
| | | 1X + rapamycin | 171 µg | 10 | | | +6% | 1.68 ± 0.37 |

The release of rapamycin into the vascular wall of a human from a nonerodible polymeric stent coating provides superior results with respect to the magnitude and duration of the reduction in neointimal hyperplasia within the stent as compared to the vascular walls of animals as set forth above.

Humans implanted with a rapamycin-coated stent comprising rapamycin in the same dose range as studied in animal models using the same polymeric matrix, as described above, reveal a much more profound reduction in neointimal hyperplasia than observed in animal models, based on the magnitude and duration of reduction in neointima. The human clinical response to rapamycin reveals essentially total abolition of neointimal hyperplasia inside the stent using both angiographic and intravascular ultrasound measurements. These results are sustained for at least one year as set forth in Table 5 below.

### Patients Treated (N=45 patients) with a Rapamycin-coated Stent

**TABLE 5.0**

| **Effectiveness Measures** | **Sirolimus FIM (N=45 Patients, 45 Lesions)** | **95% Confidence Limit** |
|---|---|---|
| Procedure Success (QCA) | 100.0% (45/45) | [92.1%,100.0%] |
| 4-month In-Stent Diameter Stenosis (%) | | |
| Mean±SD (N) | 4.8% ± 6.1 %(30) | [2.6%,7.0%] |
| Range (min,max) | (-8.2%,14.9%) | |
| 6-month In-Stent Diameter Stenosis (%) | | |
| Mean±SD (N) | 8.9% ± 7.6% (13) | [4.8%,13.0%] |
| Range (min,max) | (-2.9%,20.4%) | |
| 12-month In-Stent Diameter Stenosis (%) | | |
| Mean±SD (N) | 8.9% ± 6.1%(15) | [5.8%,12.0%] |
| Range (min,max) | (-3.0%,22.0%) | |
| 4-month In-Stent Late Loss (mm) | | |
| Mean±SD (N) | 0.00 ± 0.29 (30) | [-0.10,0.10] |
| Range (min,max) | (-0.51,0.45) | |
| 6-month In-Stent Late Loss (mm) | | |
| Mean±SD (N) | 0.25 ± 0.27 (13) | [0.10,0.39] |
| Range (min,max) | (-0.51,0.91) | |
| 12-month In-Stent Late Loss (%mm) | | |
| Mean±SD (N) | 0.11 ± 0.36 (15) | [-0.08,0.29] |
| Range (min,max) | (-0.51,0.82) | |
| 4-month Obstruction Volume (%) (IVUS) | | |
| Mean±SD (N) | 10.48% ± 2.78% (28) | [9.45%,11.51%] |
| Range (min,max) | (4.60%,16.35%) | |
| 6-month Obstruction Volume (%) (IVUS) | | |
| Mean±SD (N) | 7.22% ± 4.60% (13) | [4.72%,9.72%] |
| Range (min,max) | (3.82%,19.88%) | |
| 12-month Obstruction Volume (%) (IVUS) | | |
| Mean±SD (N) | 2.11 % ± 5.28% (15) | [0.00%,4.78%] |
| Range (min,max) | (0.00%,19.89%) | |
| 6-month Target Lesion Revascularization (TLR) | 0.0%(0/30) | [0.0%,9.5%] |
| 12-month Target Lesion Revascularization (TLR) | 0.0%(0/15) | [0.0%,18.1%] |

| | | |
|---|---|---|
| QCA = Quantitative Coronary Angiography SD = Standard Deviation IVUS = Intravascular Ultrasound | | |

Rapamycin produces an unexpected benefit in humans when delivered from a stent by causing a profound reduction in in-stent neointimal hyperplasia that is sustained for at least one year. The magnitude and duration of this benefit in humans is not predicted from animal model data.

These results may be due to a number of factors. For example, the greater effectiveness of rapamycin in humans is due to greater sensitivity of its mechanism(s) of action toward the pathophysiology of human vascular lesions compared to the pathophysiology of animal models of angioplasty. In addition, the combination of the dose applied to the stent and the polymer coating that controls the release of the drug is important in the effectiveness of the drug.

As stated above, rapamycin reduces vascular hyperplasia by antagonizing smooth muscle proliferation in response to mitogenic signals that are released during angioplasty injury. Also, it is known that rapamycin prevents T-cell proliferation and differentiation when administered systemically. It has also been determined that rapamycin exerts a local inflammatory effect in the vessel wall when administered from a stent in low doses for a sustained period of time (approximately 2 to 6 weeks). The local anti-inflammatory benefit is profound and unexpected. In combination with the smooth muscle anti-proliferative effect, this dual mode of action of rapamycin may be responsible for its exceptional efficacy.

Accordingly, rapamycin delivered from a local device platform, reduces neointimal hyperplasia by a combination of anti-inflammatory and smooth muscle anti-proliferative effects.

The anti-inflammatory effect of rapamycin is evident in data from an experiment, illustrated in Table 6, in which rapamycin delivered from a stent was compared with dexamethasone delivered from a stent. Dexamethasone, a potent steroidal anti-inflammatory agent, was used as a reference standard. Although dexamethasone is able to reduce inflammation scores, rapamycin is far more effective than dexamethasone in reducing inflammation scores. In addition, rapamycin significantly reduces neointimal hyperplasia, unlike dexamethasone.

**TABLE 6.0**

| **Group Rapamycin Rap** | **N=** | **Neointimal Area (mm²)** | **% Area Stenosis** | **Inflammation Score** |
|---|---|---|---|---|
| Uncoated | 8 | 5.24 ± 1.65 | 54 ± 19 | 0.97 ± 1.00 |
| Dexamethasone (Dex) | 8 | 4.31 ± 3.02 | 45 ± 31 | 0.39 ± 0.24 |
| Rapamycin (Rap) | 7 | 2.47 ± 0.94* | 26 ± 10* | 0.13 ± 0.19* |
| Rap + Dex | 6 | 2.42 ± 1.58* | 26 ± 18* | 0.17 ± 0.30* |

| | | | | |
|---|---|---|---|---|
| * = significance level P< 0.05 | | | | |

Rapamycin has also been found to reduce cytokine levels in vascular tissue when delivered from a stent. Rapamycin is highly effective in reducing monocyte chemotactic protein (MCP-1) levels in the vascular wall. MCP-1 is an example of a proinflammatory/chemotactic cytokine that is elaborated during vessel injury. Reduction in MCP-1 illustrates the beneficial effect of rapamycin in reducing the expression of proinflammatory mediators and contributing to the anti-inflammatory effect of rapamycin delivered locally from a stent. It is recognized that vascular inflammation in response to injury is a major contributor to the development of neointimal hyperplasia.

Since rapamycin may be shown to inhibit local inflammatory events in the vessel it is believed that this could explain the unexpected superiority of rapamycin in inhibiting neointima.

As set forth above, rapamycin functions on a number of levels to produce such desired effects as the prevention of T-cell proliferation, the inhibition of negative remodelling, the reduction of inflammation, and the prevention of smooth muscle cell proliferation. While the exact mechanisms of these functions are not completely known, the mechanisms that have been identified may be expanded upon.

Studies with rapamycin suggest that the prevention of smooth muscle cell proliferation by blockade of the cell cycle is a valid strategy for reducing neointimal hyperplasia. Dramatic and sustained reductions in late lumen loss and neointimal plaque volume have been observed in patients receiving rapamycin delivered locally from a stent. The present invention expands upon the mechanism of rapamycin to include additional approaches to inhibit the cell cycle and reduce neointimal hyperplasia without producing toxicity.

As stated previously, the implantation of a coronary stent in conjunction with balloon angioplasty is highly effective in treating acute vessel closure and may reduce the risk of restenosis. Intravascular ultrasound studies (Mintz et al., 1996) suggest that coronary stenting effectively prevents vessel constriction and that most of the late luminal loss after stent implantation is due to plaque growth, probably related to neointimal hyperplasia. The late luminal loss after coronary stenting is almost two times higher than that observed after conventional balloon angioplasty. Thus, inasmuch as stents prevent at least a portion of the restenosis process, the use of drugs which prevent inflammation and proliferation, or prevent proliferation by multiple mechanisms, combined with a stent may provide the most efficacious treatment for post-angioplasty restenosis.

Further, insulin supplemented diabetic patients receiving rapamycin-eluting stents may exhibit a higher incidence of restenosis than their normal or non-insulin supplemented diabetic counterparts. Accordingly, combinations of drugs may be beneficial.

The local delivery of drugs from a stent has the following advantages; namely, the prevention of vessel recoil and remodelling through the scaffolding action of the stent and the drugs and the prevention of multiple components of neointimal hyperplasia. This local administration of drugs to stented coronary arteries may also have additional therapeutic benefit. For example, higher tissue concentrations would be achievable than that which would occur with systemic administration, reduced systemic toxicity, and single treatment and ease of administration. An additional benefit of drug therapy may be to reduce the dose of the therapeutic compounds, thereby limiting their toxicity, while still achieving a reduction in restenosis.

Panzem® may be utilized alone or in combination with rapamycin to prevent restenosis following vascular injury. Rapamycin or sirolimus acts to reduce lymphocyte and smooth muscle cell proliferation by arresting cells in the G1 phase of the cell cycle through the inhibition of the mammalian target of rapamycin (mTOR). Rapamycin or sirolimus has shown excellent anti-restenotic effects when administered during revascularization procedures using drug-eluting stents. In recent clinical trials, the Cypher® stent, available from Cordis Corporation, which contains rapamycin or sirolimus in a polymer coating, consistently demonstrated superior efficacy against restenosis after the implantation of the stent as compared to a bare metal stent. Although the local delivery of rapamycin from a drug-eluting stent or other medical device is effective in reducing restenosis, further reductions in neointimal hyperplasia would benefit certain patient populations. Thus, the combination of rapamycin with another agent, for example, another anti-proliferative agent from a stent or other medical device may further reduce fibroproliferative vascular responses secondary to procedures involving vascular injury.

Panzem®, or 2-methoxyestradiol (2ME2) is a naturally occurring metabolite of endogenous estrogen. Its many properties provide for a wide range of potential formulations for drug delivery to treat numerous indications. Panzem® has been shown to exhibit anti-cancer activity in patients with breast cancer, prostate cancer and multiple myeloma. Panzem® is a byproduct of the metabolism of estrogen and is normally present in the body in small amounts. Panzem®, however, does not act like a hormone. Panzem® is a potent inhibitor of angiogenesis, which is what makes it such an effective anti-tumor agent. Essentially, Panzem® inhibits the formation of new blood vessels that supply oxygen and nutrients to tumor cells. Panzem® also appears to have multiple direct and indirect anti-myeloma effects as briefly described above.

Panzem®, 2-methoxyestradiol (2ME2) or methoxy- beta -estradiol is, as described above, a product of estrogen metabolism and is currently being evaluated clinically for a variety of oncologic indications. Panzem® has anti-angiogenic activity, blocks the production of vascular endothelial growth factor and directly inhibits the growth of a number of tumor cell types. Panzem® is also proapoptotic (programmed cell death) to myeloma cells. Panzem® has been found to upregulate the DR-5 receptor (of the TNF receptor family) number responsible for TRAIL-mediated apoptosis (AACR, 2003) and has microtubule stabilizing properties and reduces hypoxia-inducible factor-1 (AACR, 2003). In addition, as illustrated in detail below, Panzem® reduces human coronary artery smooth muscle cell proliferation without negatively impacting coronary artery smooth muscle cell viability.

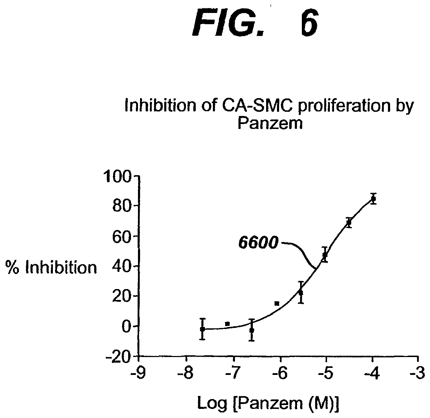

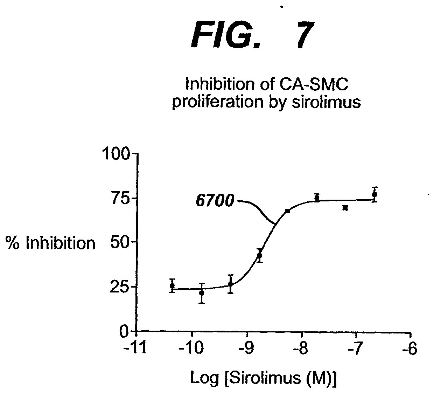

Referring to Figure 6, there is illustrated, in graphical format, the anti-proliferative activity of Panzem® in synchronized cultured human coronary artery smooth muscle cells stimulated with 2 % fetal bovine serum. As illustrated by curve 6600, Panzem® is an extremely effective inhibitor of human coronary artery smooth muscle cell proliferation *in vitro.* Figure 7 illustrates, in graphical format, the anti-proliferative activity of rapamycin or sirolimus in synchronized cultured human coronary artery smooth muscle cells stimulated with 2 % fetal bovine serum. As can be seen between a comparison between curves 6700 and 6600, both agents are effective in the *in vitro* studies.

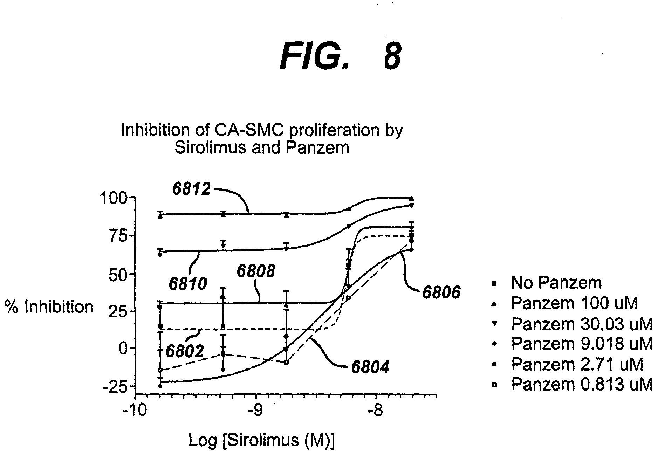

As rapamycin or sirolimus and Panzem® or other estrogen receptor modulators act to inhibit cell proliferation through different molecular mechanisms, it is possible that these agents, when combined on a drug-eluting stent, may potentiate each other's anti-restenotic activity by downregulating both smooth muscle and immune cell proliferation (inflammatory cell proliferation) by distinct multiple mechanisms. Figure 8 illustrates the potentiation of rapamycin by Panzem® on the anti-proliferative effects of rapamycin in coronary artery smooth muscle cells. This potentiation of rapamycin anti-proliferative activity by Panzem®and related compounds may translate into an enhancement in anti-restenotic efficacy following vascular injury during revascularization and other vascular surgical procedures and a reduction in the required amount of either agent to achieve the anti-restenotic effect.

Referring to Figure 8, there is illustrated, in graphical format, the anti-proliferative activity of rapamycin with varying concentrations of Panzem® in synchronized cultured human coronary artery smooth muscle cells stimulated with 2 % fetal bovine serum. The multiple curves represent various concentrations of Panzem® ranging from zero to 100 micromolar concentrations. As seen in Figure 8, the addition of Panzem® to cells treated with rapamycin increases the % of inhibition of rapamycin alone. Curve 6802 represents the response of just rapamycin. Curve 6804 represents the response of rapamycin in combination with a 0.813 micromolar concentration of Panzem®. Curve 6806 represents the response of rapamycin in combination with a 2.71 micromolar concentration of Panzem®. Curve 6808 represents the response of rapamycin in combination with a 9.018 micromolar concentration of Panzem®. Curve 6810 represents the response of rapamycin in combination with a 30.03 micromolar concentration of Panzem®. Curve 6812 represents the response of rapamycin in combination with a 100 micromolar concentration of Panzem®.

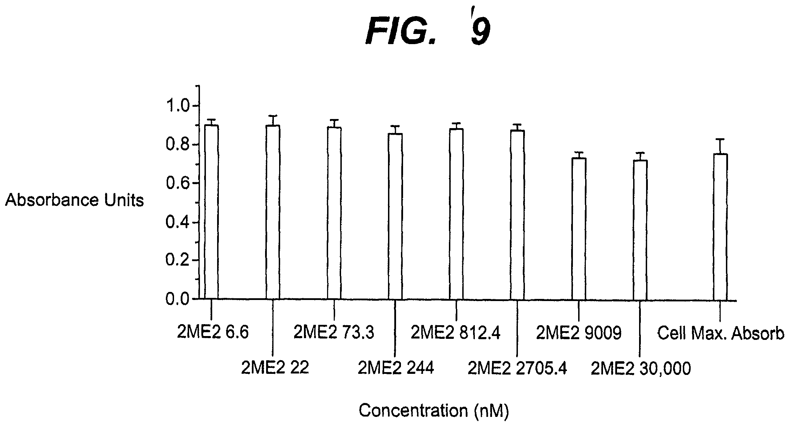

*In vitro* cytotoxicity tests or assays may be utilized to determine if drugs are potentially toxic and the level of toxicity. Essentially, *in vitro* cytotoxicity assays determine acute necrotic effects by a drug causing direct cellular damage. The idea behind these assays is that toxic chemicals affect basic functions of cells which are common to all cells. Typically, a control is utilized to determine baseline toxicity. There are a number of different assays that may be utilized. In the present description, the cytotoxicity assay utilized is based upon the measurement of cellular metabolic activity. A reduction in metabolic activity is an indication of cellular damage. Tests that can measure metabolic function measure cellular ATP levels or mitochondrial activity via MTS metabolism. Figure 9 is a graphical representation of the results of an MTS assay of Panzem®. As illustrated, concentrations of Panzem® ranging from 6.6 nanomolar to 30,000.00 nanomolar concentrations were tested without any significant fluctuations in cytotoxicity. The results of the assay indicate that Panzem® concentrations up to 30,000.00 nanomolar do not reduce human coronary artery smooth muscle cell survival.

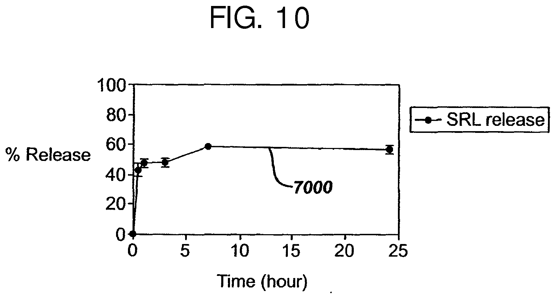

Figure 10 is a graphical representation of the *in vitro* release kinetics of rapamycin or sirolimus from a combination of rapamycin and Panzem®. In the study, the rapamycin and Panzem® are incorporated into different layers of a polymeric coating. In this study, a Bx Velocity stent is coated with a 400 µg inner layer and a 300 µg outer layer. The inner layer comprises 45 % Panzem® and 55 % EVA/BMA (50/50). The outer layer comprises 40 % rapamycin and 60 % EVA/BMA (50/50). There is no topcoat of just polymer in this study. Curve 7000 illustrates the release kinetics of rapamycin from the combination.

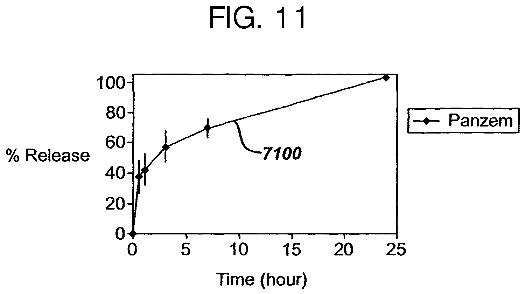

Figure 11 is a graphical representation of the *in vitro* release kinetics of Panzem® from a combination of rapamycin or sirolimus and Panzem®. In the study, the rapamycin and Panzem® are incorporated into different layers of a polymeric coating. In this study, a Bx Velocity stent is coated with a 400 µg inner layer and a 300 µg outer layer. The inner layer comprises 45 % Panzem® and 55 % EVA/BMA (50/50). The outer layer comprises 40 % rapamycin and 60 % EVA/BMA (50/50). There is no topcoat of just polymer in this study. Curve 7100 illustrates the release kinetics of Panzem® from the coating. As may be seen from a comparison of Figures 10 and 11, rapamycin elutes more slowly than Panzem® under the conditions of the test.

As is explained in more detail subsequently, a combination of incompatible polymers may be utilized in combination with rapamycin and Panzem to provide for the controlled local delivery of these drugs from a medical device. In addition, these incompatible polymers may be utilized in various combinations to control the release rates of individual drus from combinations of drugs. The correct combination of incompatible polymers may be utilized to ensure that both drugs elute at the same rate if so desired.

The exemplary stent illustrated in Figure 1 is a balloon expandable stent. Balloon expandable stents may be utilized in any number of vessels or conduits, and are particularly well suited for use in coronary arteries. Self-expanding stents, on the other hand, are particularly well suited for use in vessels where crush recovery is a critical factor, for example, in the carotid artery. Accordingly, it is important to note that any of the drugs, as well as the coatings described above, may be utilized in combination with self-expanding stents which are known in the art.

Stents may be utilized for the local delivery of drug to other areas, not immediately around the stent itself. In order to avoid the potential complications associated with systemic drug delivery, the stents of the present invention may be utilized to deliver drugs to areas adjacent the stent. For example, a rapamycin-coated stent may deliver the rapamycin to the tissues surrounding the stent as well as areas upstream and downstream of the stent. The degree of tissue penetration depends on a number of factors, including the drug, the concentration of the drug and the release rate of the drug.

The drug/carrier or vehicle compositions described above may be formulated in a number of ways. For example, they may be formulated utilizing additional components or constituents, including a variety of excipient agents and/or formulary components to affect manufacturability, coating integrity, sterilizability, drug stability and drug release rate. Within exemplary embodiments of the present invention, excipient agents and/or formulary components may be added to achieve both fast-release and sustained-release drug elution profiles. Such excipient agents may include salts and/or inorganic compounds such as acids/bases or buffer components, antioxidants, surfactants, polypeptides, proteins, carbohydrates, including sucrose, glucose or dextrose, chelating agents, such as EDTA, glutathione or other excipients or agents.

The entire stent may be coated or only a portion of the stent may be coated. The coating may be uniform or non-uniform. The coating may be discontinuous.

In accordance with the present invention, the stent may be variously configured. For example, the stent may be configured with struts or the like that form repeating geometric shapes. One skilled in the art will readily recognize that a stent may be configured or adapted to include certain features and/or to perform a certain function(s), and that alternate designs may be used to promote that feature or function.

It is generally known in the art that the application of a topcoat of a biocompatible material, for example, a polymer, may be utilized to control the elution of a therapeutic dosage of a drug combinations from a stent base coating. The basecoat generally comprises a matrix of one or more drugs and a biocompatible material such as a polymer. The control over elution results from either a physical barrier, a chemical barrier, or a combination physical and chemical barrier supplied by the topcoat material. When the topcoat material acts as a physical barrier, the elution is controlled by varying the thickness of the topcoat, thereby changing the diffusion path length for the drugs to diffuse out of the basecoat matrix. Essentially, the drugs in the basecoat matrix diffuse through the interstitial spaces in the topcoat. Accordingly, the thicker the topcoat, the longer the diffusion path and, conversely, the thinner the topcoat, the shorter the diffusion path. It is important to note that both the basecoat and the topcoat thickness may be limited by the desired overall profile of the medical device. For action as a chemical barrier, the topcoat preferably comprises a material that is less compatible with the drugs to substantially prevent or slow the diffusion, or is less compatible with the basecoat matrix to provide a chemical barrier the drugs must cross prior to being released. It is important to note that the concentration of the drugs may affect diffusion rate; however, the concentration of the drugs is dictated to a certain extent by the required therapeutic dosage as described herein. A stent may utilize a polymeric material that acts primarily as a chemical barrier for the control of elution of rapamycin from the stent. The coating may comprise a basecoat drug and polymer matrix with a topcoat that includes only a polymer. The topcoat polymer and the basecoat polymer are immiscible or incompatible, thereby creating the chemical barrier. Comparisons, however, are made with basecoat and topcoats comprising the exact same polymers or with polymers containing the same constituents in different ratios. Although the primary control mechanism is the chemical barrier, the topcoat also provides a limited physical barrier, as will be described subsequently. The basecoat may comprise any suitable fluoropolymer and the topcoat may comprise any suitable acrylate or methacrylate. In preferred embodiments, the basecoat drug/polymer matrix comprises the copolymer polyvinylidenefluoride-co-hexafluoropropylene (PVDF/HFP) as described above in detail. The copolymers utilized in this exemplary basecoat embodiment comprises vinylidenefluoride copolymerized with hexafluoropropylene in the weight ratio of 60 weight % vinyldenefluoride to 40 weight % hexafluoropropylene. The topcoat polymer may, as described above, comprise any suitable acrylate or methacrylate. Preferably, the topcoat polymer comprises poly(n-butylmethacrylate) or BMA.

PVDF/HFP and BMA are immiscible or incompatible polymers that, when mixed and precipitated from solution utilizing known techniques, will undergo phase separation. It is this incompatibility that allows a topcoat of an acrylic polymer to act as both a chemical barrier (primary mechanism) and physical barrier (secondary mechanism) to the release of a drug, such as rapamycin, from the basecoat matrix.

The combination of a PVDF/HFP basecoat and a BMA topcoat offers a number advantages over other combinations, including increased durability, increased lubriciousness and increased elution rate control. PVDF/HFP is a flexible polymer. Flexible polymers result in more durable stent coatings as they tend to move or give as the stent undergoes deformations. Poly(n-butylmethacrylate) or BMA is a more thermoplastic polymer rather than a more elastomeric polymer, and therefore more rigid than PVDF/HFP. A more rigid polymer equates to a harder surface and a harder surface is a more lubricious surface. The lubriciousness of the polymer topcoat is important during device delivery and deployment. A lubricious coating is particularly advantageous in the delivery of self-expanding stents which typically require the retraction of a delivery sheath. If the coating were not lubricious, the retraction of the delivery sheath may remove a position of the coating, including the drugs contained therein. Lubricious coatings are also advantageous for balloon expandable stents where stent/balloon separation during deployment may also remove coating. Acrylic polymers utilized in conjunction with fluoropolymers are excellent chemical and physical barriers as described above and thus provide increase elution rate control.

Although these coatings may be utilized on any number of stents as described herein, the exemplary coatings described below are utilized in conjunction with nickel-titanium self-expanding stents.

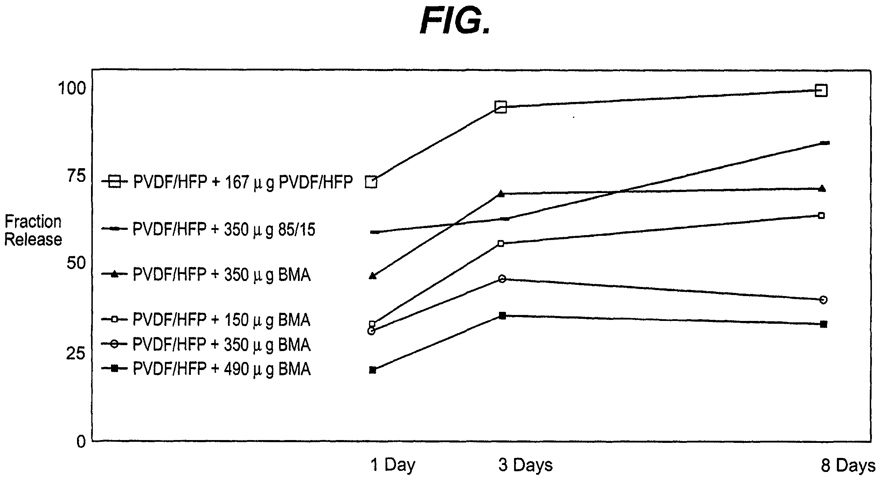

Referring now to Figure 12, there is illustrated *in vivo* drug release curves for a number of fluoropolymer/fluoropolymer and fluoropolymer/acrylic coating formulations. The *in vivo* procedure involved evaluating the elution characteristics of rapamycin-eluting stents with a number of polymer coating formulations for both the basecoat and the topcoat. Pigs are an established animal species for intravascular stent studies and accepted for such studies by the appropriate regulatory agencies. This *in vivo* study utilized male pigs of the species Sus Scrofa and strain Yorkshire pigs. S.M.A.R.T.^{™} stents, available from Cordis Corporation, were placed into the iliac and femoral arteries, PALMAZ°GENESIS°stents, available from Cordis Corporation, were placed in the renal arteries and CYPHER°stents, available from Cordis Corporation, were placed in the coronary arteries. One third of the pigs were euthanized on each of days 2, 4 and 8 and the stents and surrounding vessels were explanted and analyzed for drug content.

The data presented in Figure 12 represents the release of rapamycin *in vivo* from coated S.M.A.R.T.^{™} stents which, as described herein, are nickel-titanium stents 20 mm in length. The ratio by weight of rapamycin to polymer is 30/70 for each PVDF/HFP basecoat and 33/67 for the polyethylene-co-vinylacetate/poly(n-butylmethacrylate) (EVA/BMA) basecoat. Curve 4902 represents the elution release rate for a stent coated with a PVDF/HFP (60/40 weight ratio of VDF:HFP) and rapamycin basecoat with a 167 µg PVDF/HFP (60/40 weight ratio of VDF:HFP) topcoat. Curve 4904 represents the elution release rate for a stent coated with a PVDF/HFP (60/40 weight ratio of VDF:HFP) and rapamycin basecoat with a 350 µg PVDF/HFP (85/15 weight ratio of VDF:HFP) topcoat. Curve 4906 represents the elution release rate for a stent coated with an EVA/BMA and rapamycin basecoat (33 % EVA, 33 % BMA and 33 % rapamycin) with a 350 µg BMA topcoat. Curve 4908 represents the elution release rate for a stent coated with a PVDF/HFP (60/40 weight ratio of VDF:HFP) and rapamycin basecoat with a 150 µg BMA topcoat. Curve 4910 represents the elution release rate for a stent coated with a PVDF/HFP (60/40 weight ratio of VDF:HFP) and rapamycin basecoat with a 350 µg BMA topcoat. Curve 4912 represents the elution release rate for a stent coated with a PVDF/HFP (60/40 weight ratio of VDF:HFP) and rapamycin basecoat with a 490 µg BMA topcoat.

The data represented in Figure 12 provides an understanding of the elution rate of rapamycin from various coating combinations. A PVDF/HFP basecoat with a PVDF/HFP topcoat provides a minor physical barrier to drug elution and a minimal chemical barrier because the basecoat and topcoat are chemically identical. A topcoat of BMA on a basecoat of EVA/BMA provides a physical barrier because of the compatibility between the EVA/BMA drug matrix and the BMA topcoat chemistries. The BMA topcoat provides a slightly more effective barrier to elution because of the difference in basecoat matrix (EVA/BMA) and topcoat (BMA only) chemistries. The most substantial barrier to the elution of rapamycin, however, is observed with a PVDF/HFP basecoat matrix and a BMA topcoat because of the chemical barrier that results from the incompatible polymer chemistries. Even within the chemical barrier, however, changes in the topcoat thickness or density still provide additional levels of physical barriers to drug elution, resulting in a coating system that provides both a chemical and a physical barrier to control release of a pharmaceutical compound as indicated in curves 4908, 4910 and 4912.

The idea of utilizing incompatible polymer chemistries in conjunction with varying the thickness of the topcoat in accordance with the present invention takes advantage of what may normally be viewed as a negative aspect of chemical incompatibility to achieve a desired effect. As indicated in curve 4912, the peak elution release at 3 days is substantially less than 50 %, whereas the peak elution release at 3 days for a PVDF/HFP basecoat and a PVDF/HFP topcoat is substantially greater than 75 % as indicated in curve 4902.

Although demonstrated here with specific examples of a PVDF/HFP (60:40 weight ratio of VDF:HFP) copolymer and a BMA polymer, the concept would apply to any polymer in the family of fluoropolymers in combination with any polymer in the family of acrylics (poly(alkyl)acrylate and poly(alkyl)meth)acrylate).

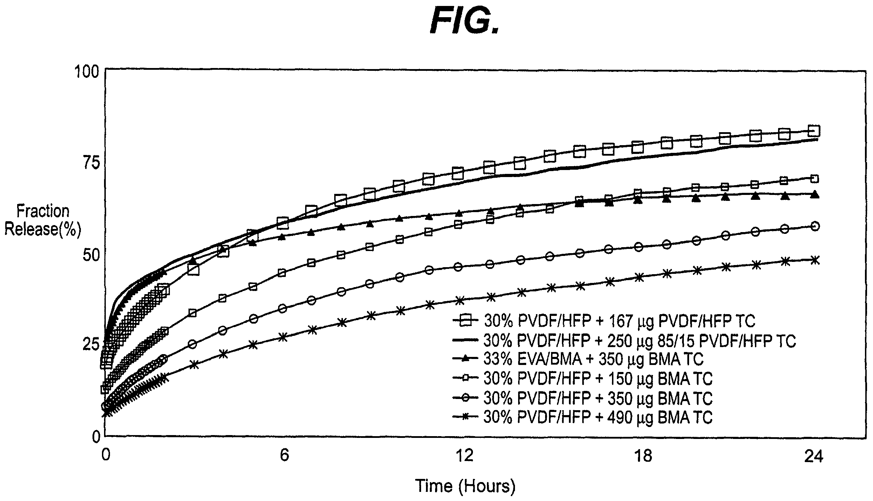

Referring to Figure 13, there is illustrated *in vitro* drug release curves for the same fluoropolymer/acrylic coating formulations described above with respect to Figure 12. In *in vitro* testing procedures, the stents are exposed to continuous flow of a surfactant medium for a period of 24 hours. The exposure of the medium causes elution of the drug (rapamycin in this instance) from the stents. The flow of medium is directed through an ultraviolet/visible spectrophotometer and the concentration of rapamycin eluting from the stent is determined as a function of time. Calculations are made based on the fraction of rapamycin released compared to the total drug content, as determined from a drug content assay on stents from the same lot.

The results from the *in vitro* testing are similar to the results from the *in vivo* testing. Essentially, a review of 5002, 5004, 5006, 5008, 5010 and 5012 indicates that once again, the most substantial barrier to the elution of rapamycin is observed with a PVDF/HFP basecoat matrix and a BMA topcoat because of the chemical barrier that results from the incompatible polymer chemistries and the physical barrier provided by the thicker topcoat as shown by curve 5012.

It is also interesting to note that a stent coated with a PVDF/HFP (60/40 weight ratio of VDF:HFP) basecoat matrix and a BMA topcoat is more durable than a stent coated with a PVDF/HFP (60/40 weight ratio of VDF:HFP) basecoat matrix and a PVDF/HFP (60/40 weight ratio of VDF:HFP) topcoat.

The design of a coated stent that elutes a drug requires the balancing of a number of design factors. For example, the addition of a coating to a stent alters the profile of the stent which in turn may have an impact on stent delivery. More specifically, the addition of a coating on a stent increases the diameter of the stent, which in turn may make delivery more difficult. Accordingly, it may be preferable to minimize the thickness of the coating while increasing the concentration of the drug. Increasing the concentration of the drug may increase its elution rate into the surrounding tissue or bloodstream. Increasing the elution rate may in turn deplete the drug, agent and/or compound prematurely. Therefore, a mechanism whereby drug concentrations may be increased while maintaining control over the elution rate and maintaining a lower profile is provided. Essentially, the chemical and physical barrier provided by the topcoat in the two layer approach provides a means for increasing drug concentrations, if preferable, maintaining a lower profile, if preferable, and maintaining more precise control over elution rates.

In addition, it is important to emphasize the multiple layers; multiple polymer approach offers the advantages of durability, flexibility and lubriciousness that a single layer approach may not be able to provide.

Vascular diseases include diseases that affect areas containing blood vessels. For example, stenosis is a narrowing or constricting of arterial lumen in a living organism (e.g. a human) usually due to atherosclerosis/coronary heart disease (CHD). Restenosis is a recurrence of stenosis after a percutaneous intervention such as angioplasty and stenting. The underlying mechanisms of restenosis comprise a combination of effects from vessel recoil, negative vascular remodelling, thrombus formation and neointimal hyperplasia. It has been shown that restenosis after balloon angioplasty is mainly due to vessel remodelling and neointimal hyperplasia and after stenting is mainly due to neo-intimal hyperplasia.

Treatment for stenosis and restenosis varies. Stenosis caused by CHD often affects quality of life and can lead to stroke, heart attack, sudden death and loss of limbs or function of a limb stemming from the stenosis. The recanalization of blood vessels may also be needed to treat individuals suffering from stenosis and restenosis. Coronary bypass can be utilized to revascularize the heart and restore normal blood flow. In other cases, balloon angioplasty may be conducted to increase the lumen size of affected areas. Overall, these treatments address the problems associated with stenosis, but they can also create the problem of restenosis that can result in recurrence of cardiac symptoms and mortality. Moreover, these treatments are not curative in nature and therefore generally are not utilized until significant disease progression has occurred.

One type of stenosis is atherosclerosis. Atherosclerosis affects medium and large arteries and is characterized by a patchy, intramural thickening that encroaches on the arterial lumen and, in most severe form, causes obstruction. The atherosclerotic plaque consists of an accumulation of intracellular and extracellular lipids, smooth muscle cells and connective tissue matrix. The earliest lesion of atherosclerosis is the fatty streak that evolves into a fibrous plaque coating the artery. Atherosclerotic vessels have reduced systolic expansion and abnormal wave propagation. Treatment of atherosclerosis is usually directed at its complications, for example arrhythmia, heart failure, kidney failure, stroke and peripheral arterial occlusion.

More particularly, atherosclerosis is a thickening and hardening of the arteries and is generally believed to be caused by the progressive build-up of fatty substances, for example cholesterol, cellular debris, inflammatory cells, calcium and other substances in the inner lining or intima of the arteries. The build-up of these substances may in turn stimulate cells in the walls of the affected arteries to produce additional substances that result in the further recruitment of cells.

Atherosclerosis is a slow, complex disease process that typically starts in childhood and progresses as the individual ages. The rate of progression may be affected by a number of factors, including blood cholesterol levels, diabetes, obesity, physical inactivity, high blood pressure and tobacco use. This build-up in commonly referred to as plaque and may grow large enough to significantly reduce blood flow through the affected arteries.

Essentially, the deposits of the various substances set forth above, and the proliferation of additional cellular substances or constituents caused thereby, substantially enlarge the intima, which in turn reduces luminal cross-sectional area of the affected arteries, which in turn reduces the oxygen supply to one or more organs. The deposits or plaque may also rupture and form thrombi that can completely obstruct blood flow in the affected artery or break free and embolize in another part of the body. If either of these events occurs, the individual may suffer a myocardial infarction, if the artery affected perfuses the heart, or a stroke, if the artery affected supplies blood to the brain. If the artery affected supplies blood to a limb or appendage, gangrene may result.

Conventional wisdom holds that myocardial infarction originates from severe blockages created by atherosclerosis. Increased deposition of lipids in the arteries and ensuing tissue reaction leads to a narrowing of the affected artery or arteries which, in turn, can result in angina and eventual coronary occlusion, sudden cardiac death or thrombotic stroke. More recent research, however, is leading to a shift in understanding atherosclerosis. Researchers now believe that at least some coronary artery disease is an inflammatory process in which inflammation causes plaque build-up or progression and rupture. These plaques which are prone to rupture, commonly referred to as vulnerable plaques, do not obstruct flow in the affected artery or arteries per se, but rather, much like an abscess, they may be ingrained in the arterial wall so that they are difficult to detect. Essentially, these vulnerable plaques cannot be seen by conventional angiography and/or fluoroscopy, and they do not typically cause symptoms of ischemia. Techniques for determining the presence of vulnerable plaques are, however, improving as discussed subsequently.

For a variety of reasons, these so-called vulnerable plaques are more likely to erode or rupture, creating emboli and exposed tissue surfaces that are highly thrombogenic. Accordingly, it is now accepted that the majority of cases of acute myocardial infarction, sudden cardiac death and thrombotic stroke result from the disruption of vulnerable atherosclerotic plaques leading to thrombosis. Therefore, these vulnerable plaques are more life-threatening than other plaques and may be responsible for as much as 60 to 80 % of all myocardial infarctions.

More specifically, unstable or vulnerable plaques are inflammatory vascular lesions that develop in atherosclerotic blood vessels. Vulnerable plaques are characterized by active inflammation, cellular hyperplasia and variable°of lumen obstruction. Morphologically, vulnerable plaques comprise a fibrous cap in contact with the lumen of the vessel overlying a core of lipid and cellular material. Vulnerable plaque lesions are not typically obstructive, in contrast to chronic stable plaques that produce ischemic symptoms. For that reason, they are not easily detected.

The hallmark of vulnerable plaques is active inflammation with significant inflammatory cell infiltration, predominantly T-lymphocytes and macrophages, causing the generation of proteolytic enzymes that essentially digest the wall of the fibrous cap, thereby inducing plaque instability and eventually plaque rupture. Plaque rupture exposes highly thrombogenic material in the lipid core to flowing blood, leading to the rapid development of occlusive thrombi. Ruptured vulnerable plaque, as stated above, is the primary cause of acute coronary and cerebral syndromes. These include unstable angina, myocardial infarction, both Q-wave and non-Q-wave myocardial infarction, cerebral stroke and transient cerebral ischemia. In other words, ruptured vulnerable plaque accounts for a significant percentage of cardiovascular morbidity and mortality.

Given the lack of currently available effective technologies for detecting vulnerable plaque, the treatment of vulnerable plaque is typically initiated only after the plaque has ruptured and clinical symptoms have developed. Detection technologies currently under investigation include refined magnetic resonance imaging, thermal sensors that measure the temperature of the arterial wall, on the premise that the inflammatory process generates heat, elasticity sensors, intravascular ultrasound, optical coherence tomography, contrast agents and near-infrared and infrared light. As better diagnostic methods evolve to identify vulnerable plaque lesions before they rupture, it becomes possible to treat discrete lesions before dangerous clinical symptoms occur. The treatment of vulnerable plaque, however, is preferably as described below.

There are two fundamental physiologic processes ongoing in active vulnerable plaque, inflammation and lipid accumulation and metabolism. Inflammation is an ongoing process which includes the inflammation of the fibrous cap and creating a cap vulnerable to rupture. Lipid metabolism is the formation of an active lipid pool or core comprising a pliable, cholesterolemic lipid material susceptible to rupture. The inflammation process is the acute phase and the lipid metabolism is the chronic phase of vulnerable plaque disease.

A stent designed to maintain vessel patency and comprising a multilaminate coating architecture that includes one or more drugs for treating both the inflammation and lipid metabolism processes may be utilized to effectively treat vulnerable plaques. In one exemplary embodiment, a stent comprising a coating having a two tier release profile may be utilized to treat both the acute and chronic phases of vulnerable plaque. The stent/drug architecture may utilize a variety of non-resorbable or resorbable polymers to control, modulate and/or optimize the delivery profile for optimal physiologic effect. In other words, specific drug delivery profiles may be utilized in conjunction with the stent to treat all aspects of vulnerable plaques.

The stent may comprise any suitable scaffold structure, including balloon expandable stents, constructed from stainless steel or other metal alloys, and/or self-expanding stents, constructed from nitinol or other shape memory metal alloys. Alternately, the stent may be made from non-metallic materials, such as ceramics and/or polymers, which may be biodegradable. The biodegradable stent would serve as a temporary scaffold and eventually dissolve over a period of time raging from days or weeks to months and years. The stent would be mounted on a delivery catheter and delivered percutaneously through the lumen of a blood vessel to the site of the vulnerable plaque lesion. The stent, as described above, is designed to maintain vessel patency and also provide structural support to the weakened or potentially weakened fibrous cap and prevent it from rupturing. The stent also provides a means for preventing further encroachment by the lesion.

Recent research has uncovered that different sex hormones may have different effects on vascular function. For example, gender differences in cardiovascular disease have largely been attributed to the protective effects of estrogen in women; premenopausal women have a lower incidence of Coronary Heart Disease. In particular, estrogen has well-known beneficial effects on lipid profile. More importantly, estrogen may directly affect vascular reactivity, which is an important component of atherosclerosis. Recent epidemiological studies suggest that hormone replacement therapy (HRT) may reduce the risk of coronary-artery disease in post-menopausal women. More particularly, many epidemiological studies suggest that estrogen replacement therapy (ERT) may be cardioprotective in postmenopausal women. The beneficial effects of these hormone therapies may also be applicable to males. Unfortunately, the systemic use of estrogen has limitations due to the possible hyperplastic effects of estrogen on the uterus and breast in women and the feminizing effects in males.

The mechanisms for these beneficial effects are probably multifactorial. Estrogen is known to favorably alter the atherogenic lipid profile and may also have a direct action on blood vessel walls. Estrogen can have both rapid and long-term effects on the vasculature, including the local production of coagulation and fibrinolytic factors, antioxidants and the production of other vasoactive molecules, such as nitric oxide and prostaglandins, all of which are known to influence the development of vascular disease.

Experimental work suggests that estrogen can also act on the endothelium and smooth muscle cells either directly or via estrogen receptors in both men and women. This appears to have an inhibitory effect on many steps in the atherosclerotic process. With respect to the interventional cardiology, estrogen appears to inhibit the response to balloon injury to the vascular wall. Estrogen can repair and accelerate endothelial cell growth *in vitro* and *in vivo*. Early restoration of endothelial cell integrity may contribute to the attenuation of the response to injury by increasing the availability of nitric oxide. This in turn can directly inhibit the proliferation of smooth muscle cells. In experimental studies, estrogen has been shown to inhibit the proliferation and migration of smooth muscle cells in response to balloon injury. Estrogen has also proved to inhibit adventitial fibroblast migration, which may in turn have an effect on negative remodelling.

Accordingly, the local or regional administration of a combination of rapamycin and 17 beta-estradiol is utilized in the treatment or stabilization of vulnerable plaque lesions. 17 beta-estradiol is chemically described as 1, 3, 5(10)-estradien-3, 17 beta-diol having the chemical notation C₁₈H₂₄O₂. 17 beta-estradiol is a natural estrogen produced in the body itself. Accordingly, there should be no biocompatibility issues when 17 beta-estradiol is administered locally.

17 beta-estradiol is generally regarded as the most potent female hormone. It is generally known that premenopausal women have a lower incidence of coronary heart diseas than other individuals and that these women produce higher levels of 17 beta-estradiol. 17 beta-estradiol has been referred to as a natural vasculoprotective agent providing a vasculoprotective effect mediated via a number of cellular mechanisms. It has been determined that 17 beta-estradiol may inhibit smooth muscle cell proliferation and migration, promote re-endothelialization and restore normal endothelial function following vascular injury. In addition, 17 beta-estradiol is known to have pleomorphic properties, i.e. the ability to occur in various distinct forms, anti-atherogenic properties, anti-inflammatory properties and antioxidant properties.

Accordingly, 17 beta-estradiol is combined with rapamycin to treat vulnerable plaque. The treatment of vulnerable plaque is achieved through the combined effect of the two drugs acting synergistically through different mechanisms to reduce smooth muscle proliferation, inflammation and atherosclerosis.

The two drugs utilized in combination with the stent would preferably prevent neointimal hyperplasia that is commonly encountered in stenting and which could lead to restenosis and device failure as described in detail above. In addition, the same drugs would preferably stabilize or passivate the lesion by reducing local inflammation and preventing further erosion of the fibrous cap. The two drugs are delivered in a polymer matrix coating applied to the stent struts and would release into the vessel wall over a predetermined period of time, preferably utilizing the dual profile release rate as briefly described above.

As set forth herein, drug-coated stents of the invention may be utilized in the treatment and/or prevention of restenosis and vulnerable plaque. The stents are coated with the two drugs as described herein.

Although shown and described is what is believed to be the most practical and preferred embodiments, it is apparent that departures from specific designs and methods described and shown will suggest themselves to those skilled in the art and may be used without departing from the scope of the invention. The present invention is not restricted to the particular constructions described and illustrated but includes all modifications that fall within the scope of the appended claims.

## Claims

1. A medical device for the treatment of atherosclerotic vulnerable plaque comprising:
a stent for maintaining vessel patency and providing structural support for a fibrous cap of the vulnerable plaque lesion;
a biocompatible polymeric coating affixed to at least a portion of the stent: and
a combination of a rapamycin and 17 beta estradiol, in therapeutic dosages, incorporated into the biocompatible polymeric coating for treating at least one of the fibrous cap and a lipid core of the vulnerable plaque lesion,
provided that a stabilizing agent which substantially hinders degradation of the pharmaceutically active agents through oxidation, other than said 17 beta estradiol, is not incorporated into the polymeric coating.

2. The medical device for the treatment of atherosclerotic vulnerable plaque according to Claim 1, wherein the stent is balloon expandable.

3. The medical device for the treatment of atherosclerotic vulnerable plaque according to Claim 1, wherein the stent is self-expanding.

4. The medical device for the treatment of atherosclerotic vulnerable plaque according to any one of Claims 1 to 3, wherein the rapamycin comprises sirolimus.

5. The medical device for the treatment of atherosclerotic vulnerable plaque according to any one of Claims 1 to 4, wherein the biocompatible coating comprises a polyfluorocopolymer comprising a first moiety selected from the group consisting of polymerized vinylidenefluoride and polymerized tetrafluoroethylene and a second moiety other than the first moiety and that is copolymerized with the first moiety,
where the second moiety is selected from the group of polymerized biocompatible monomers consisting of:
hexafluropropylene, tetrafluoroethylene, 1-hydropentafluoroproplylene, perfluoro (methyl vinyl ether), chlorotrifluoroethylene, pentafluoropropylene, trifluoroethylene, hexafluoroacetone, and hexafluoroisobutylene when polymerized vinylidenefluoride is used as the first moiety; and
hexafluropropylene, vinylidenefluoride, 1-hydropentafluoroproplylene, perfluoro (methyl vinyl ether), chlorofluoroethylene, pentafluoropropylene, trifluoroethylene, and hexafluoroacetone, hexafluoroisobutylene when polymerized tetrafluoroethylene is used as the first moiety.

## Patentansprüche

1. Medizinische Vorrichtung zur Behandlung von atherosklerotischer vulnerabler Plaque umfassend:
einen Stent zum Beibehalten der Gefäßdurchgängigkeit und Bereitstellen von struktureller Stützung für eine faserförmige Verkappung der vulnerablen Plaqueläsion;
eine biokompatible polymere Beschichtung, die an mindestens einem Teil des Stents befestigt ist; und
eine Kombination eines Rapamycins und von 17-Beta-Östradiol in therapeutischen Dosierungen, die in die biokompatible polymere Beschichtung integriert sind, zum Behandeln mindestens einer von der faserförmigen Verkappung und einem Lipidkern der vulnerablen Plaqueläsion,
vorausgesetzt, dass ein Stabilisierungsmittel, das den Abbau der pharmazeutisch aktiven Mittel durch Oxidation im Wesentlichen hindert, bei dem es sich nicht um das 17-Beta-Östradiol handelt, nicht in die polymere Beschichtung integriert ist.

2. Medizinische Vorrichtung zur Behandlung von atherosklerotischer vulnerabler Plaque nach Anspruch 1, wobei der Stent ballonexpandierbar ist.

3. Medizinische Vorrichtung zur Behandlung von atherosklerotischer vulnerabler Plaque nach Anspruch 1, wobei der Stent selbstexpandierbar ist.

4. Medizinische Vorrichtung zur Behandlung von atherosklerotischer vulnerabler Plaque nach irgendeinem der Ansprüche 1 bis 3, wobei das Rapamycin Sirolimus umfasst.

5. Medizinische Vorrichtung zur Behandlung von atherosklerotischer vulnerabler Plaque nach einem der Ansprüche 1 bis 4, wobei die biokompatible Beschichtung ein Polyfluorcopolymer umfasst, das einen ersten Anteil ausgewählt aus der Gruppe bestehend aus polymerisiertem Vinylidenfluorid und polymerisiertem Tetrafluorethylen und einen zweiten Anteil umfasst, bei dem es sich nicht um den ersten Anteil handelt, und der mit dem ersten Anteil copolymerisiert ist,
wobei der zweite Anteil aus der Gruppe von polymerisierten biokompatiblen Monomeren ausgewählt ist bestehend aus:
Hexafluorpropylen, Tetrafluorethylen, 1-Hydropentafluorpropylen, Perfluor(methylvinylether), Chlortrifluorethylen, Pentafluorpropylen, Trifluorethylen, Hexafluoraceton und Hexafluorisobutylen, wenn polymerisiertes Vinylidenfluorid als erster Anteil verwendet wird; und
Hexafluorpropylen, Vinylidenfluorid, 1-Hydropentafluorpropylen, Perfluor(methylvinylether), Chlorfluorethylen, Pentafluorpropylen, Trifluorethylen und Hexafluoraceton und Hexafluorisobutylen, wenn polymerisiertes Tetrafluorethylen als erster Anteil verwendet wird.

## Revendications

1. Dispositif médical de traitement d'une plaque vulnérable athérosclérotique comprenant:
une endoprothèse de maintien de la perméabilité d'un vaisseau et procurant un support structurel à un bouchon fibreux de la lésion de plaque vulnérable;
un revêtement polymère biocompatible apposé à au moins une partie de l'endoprothèse ; et
une combinaison d'une rapamycine et de 17 bêta oestradiol, en doses thérapeutiques, incorporée dans le revêtement polymère biocompatible pour traiter au moins l'un du bouchon fibreux et d'un noyau lipidique de la lésion de plaque vulnérable,
à condition qu'un agent stabilisant qui bloque sensiblement la dégradation des agents pharmaceutiquement actifs par oxydation, autres que ledit 17 bêta oestradiol, ne soit pas incorporé dans le revêtement polymère.

2. Dispositif médical de traitement d'une plaque vulnérable athérosclérotique selon la revendication 1, dans lequel l'endoprothèse est expansible par ballonnet.

3. Dispositif médical de traitement d'une plaque vulnérable athérosclérotique selon la revendication 1, dans lequel l'endoprothèse est auto-expansible.

4. Dispositif médical de traitement d'une plaque vulnérable athérosclérotique selon l'une quelconque des revendications 1 à 3, dans lequel la rapamycine comprend du sirolimus.

5. Dispositif médical de traitement d'une plaque vulnérable athérosclérotique selon l'une quelconque des revendications 1 à 4, dans lequel le revêtement biocompatible comprend un polyfluorocopolymère comprenant une première fraction sélectionnée dans le groupe constitué du fluorure de vinylidène polymérisé et du tétrafluoroéthylène polymérisé et une seconde fraction autre que la première fraction et qui est copolymérisée avec la première fraction,
où la seconde fraction est sélectionnée dans le groupe des monomères biocompatibles polymérisés constitués de:
l'hexafluoropropylène, du tétrafluoroéthylène, 1-hydropentafluoropropylène, perfluoro(éther de méthyl vinyle), chlorotrifluoroéthylène, pentafluoropropylène, trifluoroéthylène, de l'hexafluoroacétone, et de l'hexafluoroisobutylène lorsque du fluorure de vinylidène polymérisé est utilisé comme première fraction; et
de l'hexafluoropropylène, du fluorure de vinylidène, 1-hydropentafluoropropylène, perfluoro(éther de méthyl vinyle), chlorofluoroéthylène, pentafluoropropylène, trifluoroéthylène, et de l'hexafluoroacétone, de l'hexafluoroisobutylène lorsque du tétrafluoroéthylène polymérisé est utilisé comme première fraction.
